# EUROPEAN PATENT APPLICATION

(11) **EP 3 586 876 A1**
(43) Date of publication of application: **01.01.2020**
(21) Application number: 18178993.4
(22) Date of filing: 21.06.2018
(51) Int. Cl.: A61K 47/54

(54) **GLUCOSE-SENSITIVE PEPTIDE HORMONES**

(71) Applicant: Gubra ApS, 2970 Horsholm (DK)
(72) Inventor: Mannerstedt, Karin Margareta Sophia, DK-2100 Copenhagen Ø (DK); Jensen, Knud Jørgen, DK-2100 Copenhagen Ø (DK); Pedersen, Søren Ljungberg, 4140 Borup (DK); Lai, Johnathan, DK-1736 Copenhagen V (DK)
(74) Representative: COPA Copenhagen Patents

(57) **Abstract**

The present invention relates to a conjugate of the formula P-L-I, wherein P is a peptide hormone effecting the metabolism of carbohydrates *in vivo,* L is a hydrolysable linker molecule consisting of Lₚ and Lᵢ, and I is a molecule capable of inhibiting the effect of the peptide hormone P on the metabolism of carbohydrates *in vivo.* Under *in vivo* conditions, the conjugate is the major compound. When the concentration of glucose increases *in vivo,* the concentration of the peptide hormone effecting the metabolism of carbohydrates *in vivo* also increases.

## Description

### FIELD OF THE INVENTION

The present invention relates to glucose-responsive peptide conjugates comprising a peptide hormone affecting the metabolism of carbohydrates *in vivo,* and an agent inactivating or inhibiting the activity of the peptide hormone (or an agent facilitating inactivation or inhibition of the activity of the peptide hormone) conjugated via a hydrolysable linker molecule.

Further, the present invention relates to the use of the glucose-responsive peptide conjugates as a medicament, in particular for use as a medicament in the treatment of diabetes.

The peptide hormone part of the conjugates according to the present invention is in an inactive state in the conjugate due to the presence of the inhibitor inactivating or inhibiting the activity of the peptide hormone.

The hydrolysable linker of the conjugate facilitates the existence of the peptide hormone, the inhibitor and the conjugate in a dynamic equilibrium *in vivo.*

The present invention further relates to pharmaceutical or veterinary compositions comprising a conjugate according to the invention and at least one pharmaceutical or veterinary excipient.

In the presence of a carbohydrate, such as glucose, the peptide hormone part or the inhibitor part of the conjugate according to the invention is removed from the equilibrium when bound to the carbohydrate (although the peptide hormone-bound carbohydrate participates in a new dynamic equilibrium between the peptide hormone, carbohydrate and peptide hormone-carbohydrate conjugate), whereby the pool of non-conjugated peptide hormone parts is increased and the hormone activity increases such that the concentration of the active peptide hormone parts increases in response to increasing concentrations of glucose *in vivo.* In an alternative embodiment, glucose may catalyse the hydrolysis of the linker such that active P is formed faster. In yet another alternative embodiment, glucose may perform both catalysis and binding.

### BACKGROUND OF THE INVENTION

Peptides, in particular hormones, are frequently used as therapeutic agents to cure or manage a range of diseases. A range of therapeutic peptide hormones that have a therapeutic effect on the metabolism of carbohydrates are used in the management of a range of diseases in humans, such as diabetes, obesity and metabolic disorders.

Preferably, the activity of these peptides is needed in response to rising levels of blood glucose (i.e. rising glucose concentrations *in vivo*), and therefore a range of the therapeutic peptides affecting the metabolism of carbohydrates are to be administered after a meal, i.e. in response to rising blood glucose levels.

Such administration is cumbersome and requires frequent administrations as well as constant monitoring of the patient.

These problems could be solved by administering the peptide hormones as a depot that releases and/or activates the active peptide hormones in response to rising glucose concentrations *in vivo.*

Several technologies for achieving polypeptides with increased stability and efficacy through covalent linkage to stabilising molecules exist. Such polypeptides differ fundamentally from the glucose-responsive peptide conjugates according to the present invention in that the polypeptide conjugates according to the present invention are hydrolysable under conditions resembling conditions *in vivo* in the human body.

As an example, WO 2009/067636 A2 describes in example 12 an insulin polypeptide conjugate comprising the insulin polypeptide conjugated to PEG via a hydrazone linkage that has been reduced *in situ* to a stable hydrazide linker. The resulting polypeptide is stable and the hydrazide linkage cannot be hydrolysed *in vivo.* Insulin conjugates according to WO 2009/067636 A2 thereby differ fundamentally from the glucose-responsive peptide conjugates according to the present invention.

WO 2009/059278 A1 describes polypeptides with increased stability due to linkage to Fc-molecules. In claim 7 of this reference, a method of preparing such molecules is described. In performing that method, an intermediate acylhydrazone or hydrazone comprising an activated GLP-1 peptide and an Fc molecule are formed, which are subsequently reduced to the final stable hydrazide or hydrazine product respectively.

J. Mu et al. ("FGF21 Analogs of sustained Action Enabled by Orthogonal Biosynthesis Demonstrate Enhanced Antidiabetic Pharmacology in Rodents", Diabetes, Vol. 61, no. 2, 30 December 2011) describes FGF21 stabilised via an oxime adduct to PEG. The stable peptide conjugates according to Mu et al. thereby differ fundamentally from the glucose-responsive peptide conjugates according to the present invention.

In contrast to the above disclosures, the present invention relates to a peptide hormone effecting the metabolism of carbohydrates *in vivo,* wherein the peptide hormone is conjugated to an inactivating moiety via a hydrolysable linker molecule, whereby an equilibrium between the inactivated peptide hormone and the active peptide hormone is created *in vivo.* Thereby, e.g. a glucose-dependent insulin activity can be achieved *in vivo.*

Several technologies for achieving glucose-dependent release of insulin are known. For example, ConA (Concanavalin A) is a lectin that binds glucose and maltose. If formulated in a polymer, ConA can bind glucose during hyperglycaemic conditions leading to a swelling or breakdown of the polymer and a release of insulin (Brownlee et al., Science, 1979, 206 (4423), 1190-1191; Zion TC., 2004, PhD thesis Massachusetts Institute of Technology, "Glucose-responsive materials for self-regulated insulin delivery*"*)*.* A challenge with this method is the immunological responses to non-native ConA molecules and the stability of the ConA native molecules.

Another example is technologies based on glucose oxidase, which is highly specific for glucose and transforms glucose to oxygen, hydrogen peroxide, and gluconic acid. Formulating glucose oxidase in microgels or nanoparticles in the body will result in an acidic microenvironment during hyperglycaemic conditions, which leads to an insulin release (Gu et al., ACS Nano, 2013, 7 (8), 6758-6766; Luo et al, Biomaterials, 2012, 33, 8733-8742; Qi et al., Biomaterials, 2009, 30, 2799-2806). A challenge with the latter method is that it is cytotoxic, as hydrogen peroxide has to be quenched in the sensor. The technology has slow response rates and is susceptible to changes in pH.

Yet another example is insulins conjugated to phenylboronic acids (PBA), which can bind D-glucose through the PBA moiety. Hoeg-Jensen et al. have described such glucose-sensing insulins (Hoeg-Jensen et al., J. Pept. Sci. 2005, 11, 339-346). Boronate-insulins formulated in for example D-glucosamine polyamide polymers enable a release of insulin in the presence of glucose by displacement. Moreover, Chou et al. have reported phenylboronic acid-lipidated insulins that bind to plasma proteins, such as HSA, and in the presence of glucose, the boronic acid-insulin HSA complex will be disrupted, thus increasing the free insulin fraction in the blood (Chou et al. PNAS, 2014, 112 (8), 2401-2406). Furthermore, US005478575A discloses glycosylated insulins covalently linked to a PBA polymer. Upon increase in blood sugar, glucose competes with the glycosylated insulin for binding to the PBA polymer, thus increasing the free insulin concentration. However, a challenge with the PBA technology is the lack of specificity as it responds to all free hydroxy groups and vicinal diols as well as a higher sensitivity and selectivity for fructose compared to glycose. (Huang et al. JACS, 2013, 135, 1700-1703)

Consequently, there is a ubiquitous need in the art for new means and methods for providing peptide hormones to obtain altered, preferably increased, activity and selectivity in response to rising glucose concentrations *in vivo,* without the drawbacks mentioned above.

Accordingly, one object of the present invention is to provide means and methods for altering, preferably increasing, the activity and selectivity of a peptide hormone in response to rising glucose concentrations *in vivo* in the human or animal body.

A further object of the present invention is to provide means and methods for altering, preferably decreasing, the activity of a peptide hormone in response to falling glucose concentrations *in vivo* in the human or animal body.

A further object of the present invention is to provide means and methods for altering the activity and selectivity of a peptide hormone in response to fluctuating glucose concentrations *in vivo* in the human or animal body such that the activity of the peptide hormone decreases in response to falling glucose concentrations and increases in response to increasing concentrations of glucose *in vivo* in the human or animal body.

Further, an object of the present invention is to provide glucose-responsive therapeutic peptide conjugates, in particular glucose responsive insulins (GRI).

### DEFINITIONS:

According to the present invention, peptide conjugates are conjugates comprising a first part comprising a peptide hormone and a second part comprising an inactivating means, i.e. a means for inactivating the peptide hormone herein also referred to as an "inhibitor", the first and the second part being conjugated via a hydrolysable linker moiety.

According to the present invention, peptide hormones are peptides that activate or inactivate certain molecular pathways *in vivo,* whereby the metabolic activity of a subject to which the peptide hormone is administered is altered. Preferably, peptide hormones according to the present invention include pancreatic hormones, such as insulin or amylin, gut hormone such as glucagon-like peptide-1 (GLP-1), gastric inhibitory polypeptide (GIP, also known as glucose-dependent insulinotropic peptide) or cholecystokinin (CCK), adipocyte-derived hormone such as adiponectin or leptin, myokines such as interleukin 6 (IL-6) or interleukin 8 (IL-8), liver-derived hormone such as betatrophin, fibroblast growth factor 19 (FGF19) and fibroblast growth factor 21 (FGF21). Further, the peptide hormones according to the present invention may be brain-derived proteins such as brain-derived neurotrophic factor (BDNF) and growth hormones. Peptide hormones to which glucose is bound are also comprised by the definition of P.

According to the present invention, an insulin analogue is a peptide having an insulin-like function *in vivo* in the human or animal body, i.e. a function in the regulation of the metabolism of carbohydrates, fats and proteins by promoting the absorption of especially, glucose from the blood into fat, liver and skeletal muscle cells.

According to the present invention a masked aldehyde or a masked ketone is a functional group that is cleaved upon hydrolysis into a compound comprising an aldehyde or a ketone moiety respectively, in addition to another chemical entity.

According to the present invention the term "catalyse" has the usual meaning in the art. Hence, a reaction may be catalysed by a catalyst, which is not consumed in the catalysed reaction. The catalyst accelerates the reaction rate without affecting the equilibrium (i.e. both the forward and reverse reaction rates are increased such that the equilibrium remains constant). Put in another way, a catalyst may lower the energy barrier (activation energy) for the reaction. In the present context, glucose may act as a catalyst. Furthermore, a catalyst may be needed in a certain concentration before effective and/or observable catalysis takes place. Hence, a threshold concentration (e.g. a certain glucose concentration) is needed for effective and/or observable catalysis to take place.

According to the present invention, the term "dynamic equilibrium" is the state in a reversible reaction in which both reactants and products are present in concentrations that have no further tendency to change with time so that there is no observable change in the properties of the system. The reaction rates of the forward and backward reactions are generally not zero, but equal. Thus, there are no net changes in the concentrations of the reactant(s) and product(s).

According to the present invention, a hydrolysable linker means a compound that binds the peptide hormone and the inhibitor together, but that is prone to a certain extent of hydrolysis under *in vivo* conditions such that the majority of the peptide hormone part of the conjugate is present in association with the inhibitor, i.e. as part of the peptide conjugates according to the invention, under *in vivo* conditions (at normal blood glucose levels), and a minority of the peptide hormone part of the conjugate is present free of the linker compounds under *in vivo* conditions (at normal blood glucose levels). According to the present invention, a linker is hydrolysable *in vivo* if the linker (concentration 0.42 mM) hydrolyses *in vitro* in phosphate buffer pH 7.4 such that an equilibrium between linker and hydrolysed linker exists within 5 hours such that at least 1% and up to 50% of the linker is hydrolysed.

According to the present invention, at least one of the conjugate parts P-Lₚ and Lᵢ-I binds covalently to glucose *in vivo,* if the hydrolysed linker, under conditions as described in example 6 (linker was dissolved (concentration 0.42 mM) in 5% MeCN or 10% DMSO in PBS buffer at pH 7.4 and 37 °C, with 10000 eq. of glucose added immediately after solvation), produce a conjugate between glucose and at least part of the linker within 96 hours, preferably within 72 hours, more preferably within 24 hours.

According to the present invention the hydrolysis of the hydrolysable linker L is being promoted by glucose if the linker (concentration 0.42 mM) hydrolyses *in vitro* in phosphate buffer pH 7.4 in the presence of 10.000 equiv. glucose such that an equilibrium between linker and hydrolysed linker exists within 5 hours such that at least 2% and up to 100% of the linker is hydrolysed and such that the amount of hydrolysed linker is increased by increasing the presence of glucose.

According to the present invention, the inactivator (I) is a molecule capable of inactivating the active site of a peptide (P). Such molecule (I) may be e.g. a molecule capable of limiting the exposure of the active site of P to the environment. As an example, limiting the exposure of the active site of P to the environment may e.g. be achieved by covalently binding P (via the hydrolysable linker) to macromolecular substances such as PEG, Fc antibody, XTEN, PASylation, serum albumin, carbohydrate polymers (such as dextran, HES, polysialylation), nanoparticles and hydrogels. Alternatively, I may be small molecule albumin binders or lipids capable of non-covalent binding serum proteins such as serum albumin. Thus, the inhibitor or inactivator (I) may be a molecule capable of non-covalent binding to larger protein structures in human serum, thereby facilitating the clustering of multiple conjugates according to the invention *in vivo.* Hence, the inhibitor or inactivator (I) may be a small molecule albumin binder or a lipid molecule, or any molecule capable of non-covalent binding to serum albumin. An inactivator or inhibitor of insulin may also be a molecule that is bound, linked via (L) to insulin at a position that inhibits the activity of insulin. According to the present invention, the inactivator (I) is a molecule capable of inactivating a peptide (P), if, under conditions as described in example 6 (where P is insulin), the activity of PI is 50% or less of the activity of P.

According to the present invention, C₁-C₁₀ alkyl is to be understood as univalent groups derived from alkanes (C*ₙ*H_{2*n*+2}) or cycloalkanes (C*ₙ*H_{2*n*}) by removal of a hydrogen atom from any carbon atom where n is 1-10, i.e. 1-10 carbon atoms are comprised. C₁-C₁₀ alkyls may be linear (-C*ₙ*H_{2*n*+1}), branched (-C*ₙ*H_{2*n*+1}) or cyclic (-C*ₙ*H_{2*n*-1}). Cₓ-C_{y}, such as C₁-C₁₀, generally refers to the total number of carbon atoms also for alkenyls, alkynyls, alkylene, alkenylene and alkynylene. C₂-C₁₀ alkenyls and alkynyls may be linear or branched and C₅-C₁₀ alkenyls may be cyclic. Furthermore, C₂-C₁₀ alkenyls or alkynyls may contain one or more alkene(s) or alkyne(s).

According to the present invention, alkylene is to be understood as an alkanediyl group not necessarily having the free valencies on adjacent carbon atoms, such as propane-1,3-diyl (-CH₂CH₂CH₂-) or such as propane-1,2-diyl (-CH(CH₃)CH₂-). Alkenylene and alkynylene should be understood in a similar context as an alkenediyl or alkynediyl comprising at least one double bond (alkene) or triple bond (alkyne) respectively.

According to the present invention, a Cₓ-C_{y} lipid molecule, such as C₁₂-C₂₆, generally refers to the total number of carbon atoms in the lipid molecule. In the present context, a lipid molecule may be either hydrophobic or amphiphilic and include but are not limited to fatty acids, glycerolipids, glycerophospholipids, cholesterol and analogues of any thereof.

According to the present invention, aryl and heteroaryl are groups derived from arenes or heteroarenes by removal of one or more hydrogen atoms from a ring atom. Thus, in the present context an aryl or heteroaryl may have multiple free valencies. Furthermore, arenes has the usual meaning in the art as being mono- or polycyclic aromatic hydrocarbons. Likewise, heteroarenes are heterocyclic compounds formally derived from arenes by replacement of one or more methine (-C=) and/or vinylene (-CH=CH-) groups by trivalent or divalent heteroatoms, respectively, in such a way as to maintain the continuous n-electron system characteristic of aromatic systems and a number of out-of-plane n-electrons corresponding to the Hückel rule (4 n + 2). A heteroatom has the usual meaning in the art as being an atom that is not carbon (C) or hydrogen (H). Typical examples of heteroatoms include but are not limited to nitrogen (N), sulfur (S), oxygen (O), and phosphorus (P).

According to the invention, a 5, 6, 9 or 10- membered aryl or heteroaryl includes but are not limited to the group consisting of moieties derived from benzene, naphthalene, pyrrole, furane, thiophene, thiazole, isothiazole, oxazole, isooxazole, pyrazole, imidazole, 1,2,3-oxadiazole, 1,2,4-oxadiazole, 1,2,5-oxadiazole, 1,3,4-oxadiazole, 1,2,3-triazole, 1,2,4-triazole, pyridine, pyridazine, pyrimidine, pyrazine, 1,2,4-triazine, 1,3,5-triazine, 1H-indole, indolizine, 1H-indazole, benzimidazole, 4-azaindole, 5-azaindole, 6-azaindole, 7-azaindole, 7-azaindazole, pyrazolo[1,5-a]pyrimidine, benzofuran, isobenzofuran, benzo[b]thiophene, benzo[c]thiophene, benzo[d]isoxazole, benzo[c]isoxazole, benzo[d]oxazole, benzo[c]isothiazole, benzo[d]thiazole, benzo[c][1,2,5]thiadiazole, 1H-benzotriazole, quinolone, isoquinoline, quinoxaline, phthalazine, quinazoline, cinnoline, 1,8-naphthyridine, pyrido[3,2-d]pyrimidine, pyrido[4,3-d]pyrimidine, pyrido[3,4-b]pyrazine, and pyrido[2,3-b]pyrazine.

According to the present invention, EWG means electron withdrawing groups and EDG means electron donating groups. Typical EWG includes but are not limited to triflyl (-SO₂CF₃), trihalides (-CF₃, CCl3), cyano (-CN), sulfonates (-SO₃H), nitro (-NO₂), ammonium (-NH₃⁺), quaternary amine (-NR₃⁺), aldehyde -(CHO), ketones (-COR), carboxylic acid (-COOH), acyl chloride (-COCl), esters (-COOR), amide (-CONH₂) and halides (-F, -Cl, -Br, -I). Typical EDG includes but are not limited to primary, secondary and tertiary amines (-NH₂, -NHR, -NR₂), ethers (-OR), hydroxy (-OH), amides (-NHCOR), esters (-OCOR), alkyls, phenyl and vinyl. Electron donating groups and electron withdrawing groups are a result of mesomeric effects as a result of p-orbital overlap (resonance) as well as inductive effects through σ-bonds due to relative electronegativity between atoms.

According to the present invention, a molecule capable of inactivating the active site of a peptide (P) is a molecular structure which, when present in the conjugate, is responsible for decreasing the activity of the relevant peptide hormone to an extent that the activity of the relevant peptide hormone is reduced to less than 50%, preferably less than 40%, even more preferably less than 30%, even more preferably less than 20%, and most preferably to less than 10% of the activity of the peptide (P) (i.e. the activity of P in the absence of the molecule capable of inactivating the active site of a peptide (P)) under *in vitro* conditions as described in example 7 (where P is insulin). The inhibition capability of the inactivator or inhibitor may be measured using a functional receptor assay for the peptide (P). First, the functionality (EC50) of the P-L-I molecule dissolved in PBS, pH 7.4, could be measured, and secondly, the P-Lₚ-Glc or P-Lₚ molecule could be measured (if relevant in the presence of a relevant macromolecular structure). P-Lₚ-Glc could be formed by adding 10.000 equivalents glucose to a P-L-I mixture, dissolved in PBS pH 7.4, and left to react for 72 h. If the inhibitor (I) is a molecule capable of non-covalent binding to larger protein structures in human serum, such as a molecule capable of binding to a plasma protein, the relevant structure or protein should be included in the experiment. The functionality (EC50) of P-L-I compared to P-Lₚ-Glc determines the inhibitor (I) ability to decrease the activity of the peptide (P).

### SUMMARY OF THE INVENTION

The peptide conjugates according to the present invention address and solve the problem of altering the hormonal activity of a peptide hormone in response to fluctuating carbohydrate concentrations *in vivo* by creating a dynamic equilibrium releasing active peptide hormones in response to rising glucose concentrations *in vivo.* In response to falling glucose concentrations *in vivo,* the pool of active peptide hormones is decreased due to less and/or slower release from the pool of conjugated peptides, and a relatively short half-life of the peptide hormone itself.

The invention thus provides new methods and means for providing glucose-responsive therapy. The therapeutic peptide conjugates according to the invention are glucose-responsive by consisting of a first part comprising an active peptide hormone, which is coupled to a second part comprising an inactivating means. The inactivation means may inactivate the peptide hormone by e.g. facilitating depot formation, facilitating binding to large molecules, such as serum albumin, or by directly inhibiting the active site of the peptide hormone. Conjugates consisting of a first part comprising a peptide hormone coupled to a second part comprising means that inactivate the peptide hormone are known in the art, i.e. as insulin depots wherein insulin is covalently or non-covalently coupled to larger molecules such as serum albumin. These insulin depots slowly and constantly deliver insulin to the body *in vivo.*

The present invention resides e.g. in the use of a linker comprising a 5-membered cyclic masked aldehyde or 5-membered cyclic masked ketone, that is hydrolysable *in vivo* to a certain extent (i.e. dynamic equilibrium) to associate the peptide hormone and the inactivating means, where the hydrolysable linker (or a part thereof) is capable of covalently binding a carbohydrate, preferably glucose, after hydrolysis. Thus, re-association after hydrolysis is prevented by the presence of a carbohydrate, preferably glucose. In an alternative embodiment, the presence of the carbohydrate, preferably glucose, prevents the reformation of the linker (L), after the hydrolysis of L, through another mechanism. In yet an alternative embodiment, the presence of the carbohydrate, preferably glucose, catalyses the hydrolysis of L.

The first and the second parts of the conjugate of the invention are linked via a hydrolysable linker. At least one part of the hydrolysable linker binds glucose after being hydrolysed, or alternatively, glucose promotes the hydrolysis of the hydrolysable linker.

In solution, e.g. *in vivo,* the conjugates according to the invention will be present in a dynamic equilibrium comprising the inactive peptide conjugate (where the linker is unhydrolysed) as well as the two parts thereof in isolation, i.e. the active peptide hormone, where the linker is hydrolysed, and the inactivation means in isolation.

When glucose is present, glucose will bind to at least one part of the hydrolysable linker, whereby the glucose-bound part, i.e. the glucose-bound active peptide hormone or the glucose-bound inactivating means, will no longer take part in the dynamic equilibrium between P-L-I, PL_{P} and L_{I}I.

The dynamic equilibrium will replace the removed parts and thereby deliver new active peptide hormones when the glucose concentration increases. Put in another way, the dynamic equilibrium will alter the concentrations of reactants and products such that the equilibrium constant remains constant.

In an alternative embodiment, when glucose is present, glucose promotes the hydrolysis of the hydrolysable linker, whereby the dynamic equilibrium is altered such that an increased amount of active peptide hormone is formed in the dynamic equilibrium.

### DETAILED DESCRIPTION OF THE INVENTION

The present invention is based on the inventive finding that peptide hormones, and the activity thereof, can be made responsive to glucose concentrations *in vivo* by coupling the peptide hormones to an inactivating means via an *in vivo* hydrolysable linker L comprising a 5-membered cyclic masked aldehyde or 5-membered cyclic masked ketone that binds glucose when hydrolysed, or the hydrolysis of which is promoted by glucose.

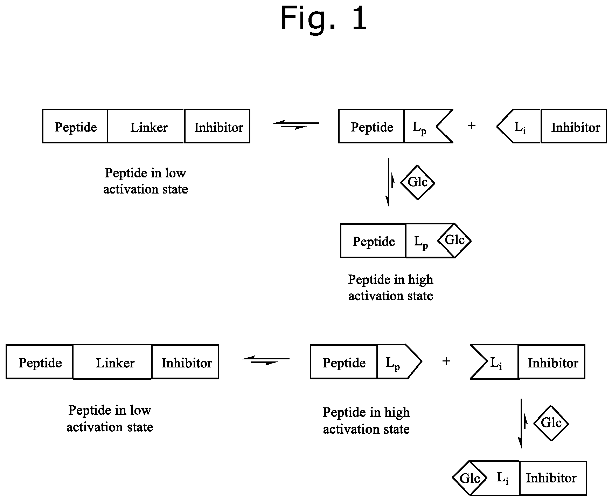

Thereby, a dynamic equilibrium exists *in vivo* between the active peptide hormone and the inactivated peptide conjugate as illustrated in Figure 1 (merely an example). In the absence of glucose (Glc), or in the presence of very low concentrations of glucose, the majority of the peptide hormones will be in the form of peptide conjugates according to the invention, i.e. they will be in the inactivated form due to the dynamic equilibrium favouring the inactivated conjugate.

However, in the presence of glucose, glucose binds to the active peptide hormone or the inactivation agent, whereby the formation of inactivated peptide conjugate from that peptide hormone or inactivation agent, to which glucose is bound, is hindered (i.e. the peptide hormone or inactivation agent, to which glucose is bound, is prevented in participating in the dynamic equilibrium with P-L-I). In such a situation, the dynamic equilibrium will react towards release of active peptide hormone from the reservoir of inactive peptide conjugates such that the equilibrium constant remains constant in accordance with Le Chatelier's principle. In other words, the presence of glucose will initiate the release of active peptide hormones from the reservoir of peptide hormones being present as part of an inactive peptide conjugate. Falling concentrations of glucose will initiate decreased levels of active peptide hormones. As another alternative, the same effect may be achieved by the presence of the carbohydrate, preferably glucose, preventing the reformation of the linker (L) after the hydrolysis of L by any other mechanism. In yet another alternative, the presence of the carbohydrate, preferably glucose, catalyses the hydrolysis of the linker such that the reaction rate of hydrolysis is increased. This catalysis may require a certain threshold concentration of the carbohydrate, preferably glucose, such that no or little catalysis is present at normal glucose concentration, whereas catalysis is present at higher glucose concentration. Put in another way, at normal blood glucose concentration, the active peptide hormone is not or only slowly released, even if the dynamic equilibrium is not yet reached, whereas at higher blood glucose concentration the active peptide hormone is released or released faster towards equilibrium due to catalysis. Alternatively, the same effects may be achieved by the hydrolysis of the hydrolysable linker being promoted by glucose. In the present context, the term "promoted" (i.e. the hydrolysis of the hydrolysable linker being promoted by glucose) should be understood in the broadest sense as any mechanism or means aiding in the release of active peptide P-Lp in response to rising glucose concentration.

This finding paves the way for e.g. producing glucose-responsive depots of peptide hormones, such as glucose-responsive depots of insulin.

Accordingly, in a first aspect, the present invention relates to a conjugate of the formula P-L-I, wherein P is a peptide hormone effecting the metabolism of carbohydrates *in vivo,* L is a hydrolysable linker molecule consisting of Lp and Li, and I is a C₁₂-C₂₆ lipid molecule capable of inactivating or inhibiting the effect of the peptide hormone P on the metabolism of carbohydrates *in vivo,* characterised in that
a. the linker molecule L comprises a 5-membered cyclic masked aldehyde or 5-membered cyclic masked ketone hydrolysable *in vivo,* such that the conjugate P-L-I and the conjugate parts P-Lp and Li-I exist in a dynamic equilibrium *in vivo* where the conjugate P-L-I exists in molar excess of at least one of the conjugate parts P-Lp and Li-I, and further characterised in that
b. at least one of the conjugate parts P-Lp and Li-I binds covalently to glucose, whereby the concentration of P that is not bound to I increases *in vivo* when the concentration of glucose increases *in vivo,* or, alternatively further characterised in that the hydrolysis of the hydrolysable linker L is being promoted by glucose.

### P:

P is a peptide hormone effecting the metabolism of carbohydrates in vivo.

In one embodiment of the invention, the peptide hormone P is a pancreatic hormone, such as insulin or amylin. In another aspect of the invention, the peptide hormone is a gut hormone, such as glucagon-like peptide-1 (GLP-1), gastric inhibitory polypeptide (GIP, also known as the glucose-dependent insulinotropic peptide or cholecystokinin (CCK) or analogues thereof. In another embodiment of the invention, the peptide hormone is an adipocyte-derived hormone such as adiponectin or leptin. In another embodiment of the invention, the peptide hormone is a myokine such as interleukin 6 (IL-6) or interleukin 8 (IL-8) or analogues thereof. In another embodiment of the invention, the peptide hormone is a liver-derived hormone such as betatrophin, fibroblast growth factor 19 (FGF19) or fibroblast growth factor 21 (FGF21) or analogues thereof. In another embodiment of the invention, the peptide hormone is a brain-derived protein, such as brain-derived neurotrophic factor (BDNF) or analogues thereof. In another embodiment of the invention, the peptide hormone is a growth hormone or an analogue thereof.

In a preferred embodiment of the invention, the peptide hormone (P) is insulin or an insulin analogue or a molecule capable of activating the insulin receptor (INR). Preferably, P is capable of activating the insulin receptor below µM concentrations, such as at a concentration of less than 1 µM. In a highly preferred embodiment of the invention, P is insulin or an insulin analogue. According to the present invention, peptide hormones to which glucose is bound are also comprised by the definition of P.

### I:

I is a molecule or substance that is capable of inactivating or inhibiting the effect of the peptide hormone P on the metabolism of carbohydrates *in vivo* e.g. by facilitating inactivation or inhibition of the activity of the peptide hormone by formation of inactive complexes *in vivo* or by direct inhibition of the active site of the peptide hormone.

Molecules and mechanisms capable of inactivating or inhibiting the effect of the peptide hormone P on the metabolism of carbohydrates *in vivo* are well-known in the art. Inactivating or inhibiting a peptide hormone *in vivo* may, in general, be achieved by limiting the exposure of the active site of P to the environment. As an example, limiting exposure of the active site of P to the environment may e.g. be achieved by covalent binding P (via the hydrolysable linker part of the conjugate) to macromolecular substances such as PEG, Fc antibody, XTEN, PASylation, serum albumin, carbohydrate polymers (such as dextran, HES, polysialylation), nanoparticles and hydrogels. Alternatively, I may be small molecule albumin binders or lipids capable of non-covalent binding to serum albumin. An inactivator or inhibitor of insulin may also be a molecule that is bound, linked via L, to insulin at a position that inhibits the activity of insulin.

In an embodiment of the invention, I is a large molecule, such as serum albumin covalently linked to the hormone peptide (P) via the hydrolysable linker (L).

In another embodiment, I may be an agent capable of clustering multiple components in structures, such as hydrogels or nanoparticles. Thus, in another embodiment of the invention, I is a hydrogel. A hydrogel is a hydrophilic gel that consists of a network of polymer chains in which water is the dispersion medium. In this embodiment of the invention, the hydrogel is the inhibitor (I), and chemical handles on the hydrogel allow for covalent attachment of the peptide hormone (P) via the linker (L).

In another embodiment of the invention, I is a nanoparticle. Nanoparticles (which may be viewed as a type of colloidal drug delivery system) comprise particles with a size range from 2 to 1000 nm in diameter. In this embodiment of the invention, the nanoparticles may be coated with a polymer allowing covalent attachment of the peptide hormone (P) via the linker (L).

In a highly preferred embodiment of the invention, I is an agent capable of inhibiting the active site of P, e.g. an inhibitor that is bound to the peptide hormone (e.g. insulin) at a position that inhibits the activity of the peptide hormone. This may be done e.g. by linking I to amino acid A1 in insulin.

In another highly preferred embodiment of the invention, I is an agent capable of clustering multiple conjugates of the formula P-L-I *in vivo.*

In yet another highly preferred embodiment of the invention, I is an agent capable of inactivating or inhibiting P by facilitating depot formation, e.g. by facilitating non-covalent binding of P-L-I to large molecules, such as serum albumin. In such an embodiment, I may inhibit the conjugate itself (i.e. prior to binding) or inhibition may first be obtained upon non-covalent binding of P-L-I to the large molecule, such as serum albumin.

In yet another highly preferred embodiment of the invention, I is an agent capable of non-covalently binding to serum albumin, such as fatty acids or small molecule albumin binders, or other plasma proteins. Preferably, such agent is a fatty acid which comprise the structure selected among A1-7; 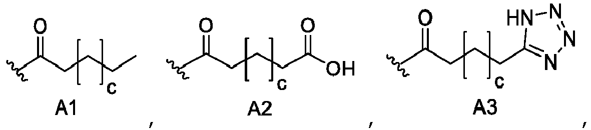 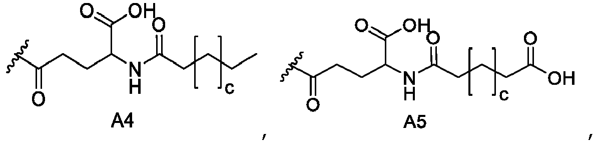 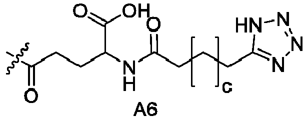 or 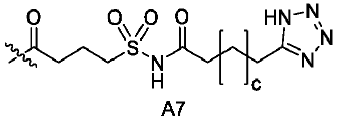 and c is at least 8, preferably at least 10, more preferably at least 12. The optimal chain length depends on the choice of A. Thus, in a preferred embodiment where I comprise A1, c is preferably 14. In another preferred embodiment, where I comprise A2, c is preferably 12.

In an even more preferred embodiment of the invention, the inhibitor I is selected among 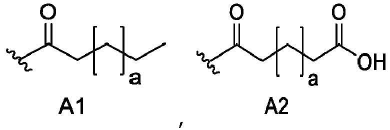 or 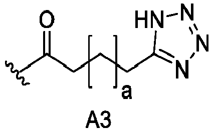 wherein a is an integer from 8-22, preferably 10-20, more preferably 12-16.

### Other preferred inhibitors (I):

In the present invention inhibitors may serve several purposes in the conjugates of the invention, besides inhibiting the peptide hormone (P). Thus, the inhibitor may also serve to alter the pharmacokinetic (ADME) properties of the conjugates, such as lowering metabolism and excretion. Thus, in another highly preferred embodiment, I is a large molecule that prevents the conjugated peptide from being cleared in the kidney. Such molecules may be recombinant albumin, Fc antibody, PEG, or carbohydrate polymers, such as dextran, hydroxyethyl starch (HES) or a polymer of sialic acids (polysialylation).

In addition to inhibiting the activity of the hormone P in the conjugate, recombinant albumins are able to load peptides (P) via the linker (L) leading to low renal excretion of the peptide hormone P, providing a system that is longer lasting *in vivo.* Similarly, conjugating the peptide (P) via the linker (L) to the Fc part of the IgG antibody enables recycling of the conjugate via the Fc receptor leading to low renal clearance. In the same way, chemical conjugation of the peptide (P) via the linker (L) to polyethylene glycol (PEG), using PEG20 to PEG80, prevents renal excretion by increasing the hydrodynamic volume of the peptide. Accordingly, in one highly preferred embodiment of the invention, I is a recombinant albumin, Fc antibody or PEG.

In the same way, carbohydrate polymers, such as dextran, hydroxyethyl starch (HES) or polysialylated conjugates thereof, may prevent the conjugated peptide from being cleared in the kidney. Dextran polymers may be obtained from bacteria such as *L. mesenteroides* and are D-glucose polymers linked by α(1-6) glycosidic linkages and a small extent of α(1-3) bonds (∼95% α(1-6) and 5% α(1-3) in the case of *L. mesenteroides*). In addition to unmodified dextran, various synthetic dextran derivatives, such as carboxymethyl-dextran (CMD), diethylaminoethyl dextran (DEAED), glycosylated versions of CMD such as galactose-CMD (Gal-CMD) and mannose-CMD (Man-CMD), carboxymethyl benzylamide dextran (DCMB), carboxymethyl sulfate dextran (DCMSu), and carboxymethyl benzylamide sulfate dextran (DCMBSu) can be used to chemically modify a peptide (P) via the linker (L). Dextran, as PEG, increases the hydrodynamic volume of the peptide leading to a reduced renal filtration. Hydroxyethyl starch (HES) is a modified natural polymer obtained by controlled hydroxyethylation of the plant polysaccharide amylopectin. Amylopectin is a polymer of D-glucose containing primarily α-1,4 glycosidic bonds, but also a lower abundance of α-1,6 linkages, leading to a naturally branched carbohydrate. Hydroxyethylation of the starch precursor serves two purposes: first, to increase the water solubility by increasing the water-binding capacity and decreasing viscosity, and second, to prevent immediate degradation by plasma α-amylase and subsequent renal excretion. HES can be chemically modified in the reducing end allowing for the attachment of the P-L-moiety. 'Sialic acid' does not refer to a single chemical entity, but rather to an entire group of nine carbon monosaccharides, the most important examples being 5-N-acetylneuraminic acid (Neu5Ac), 5-N-glycolylneuraminic acid (Neu5Gc), and 2-keto-3-deoxynonulosonic acid (Kdn). However, the only observed polysialic acid (PSA) variant in humans is colominic acid (CA), the linear α-2,8-linked homopolymer of Neu5Ac. Conjugation of polysialic acids to peptides or proteins is referred to as polysialylation. Similar to PEG and the PEG mimetics dextran and HES, the driving force behind the long pharmacokinetic profile of polysialylated conjugates is thought to be an increase in hydrodynamic radius, resulting in decreased renal clearance as well as shielding from enzymatic degradation and antibody recognition.

When present in the conjugate, an inactivator or inhibitor according to the present invention should be responsible for decreasing the activity of the relevant peptide hormone to an extent that the activity of the relevant peptide hormone is reduced to less than 50%, preferably less than 40%, even more preferably less than 30%, even more preferably less than 20%, and most preferably to less than 10% of the activity in the absence of the attached inactivator or inhibitor under in vitro conditions. The inhibition capability of the inactivator or inhibitor may be measured using a functional receptor assay for the peptide (P). First, the functionality (EC50) of the P-L-I molecule dissolved in PBS, pH 7.4, could be measured, and secondly, the P-Lp-Glc molecule could be measured (if relevant in the presence of a relevant macromolecular structure). P-Lp-Glc could be formed by adding of 10.000 equivalents glucose to a P-L-I mixture, dissolved in PBS pH 7.4, and left to react for 72 h. If the inhibitor (I) is a molecule capable of binding to a plasma protein, the protein should be included in the experiment. The functionality (EC50) of P-L-I compared to P-Lp-Glc determines the inhibitor (I) ability to decrease the activity of the peptide (P).

### L:

L is a 5-membered cyclic masked aldehyde or 5-membered cyclic masked ketone hydrolysable *in vivo* consisting of Lp and Li. When L is hydrolysed, a molecule of water is added to L, which results in the fragmentation of L into Lp and Li.

L must be hydrolysable *in vitro* and *in vivo,* but preferably L is only hydrolysed at a low frequency, such that L, Lp and Li exist in a dynamic equilibrium in water under *in vitro* and *in vivo* conditions, wherein L (the conjugate) is the major compound and Li and Lp (the conjugate parts) are the minor compounds. In other words, L exists in molar excess of Lp and Li under *in vitro* conditions, meaning that P-L-I exists in molar excess of P-Lp and Li-I under *in vitro* conditions. A linker is said to be hydrolysable according to the present invention if it results in the existence of a dynamic equilibrium under in vitro conditions as described in example 6, in which P-L-I exists in molar excess with regard to the presence of the conjugate parts P-Li and/or Lp-I of at least 2:1, preferably at least 3:1, more preferably at least 4:1, even more preferably at least 5:1, even more preferably at least 10:1, even more preferably at least 50:1, and most preferably at least 100:1. The in vitro hydrolysability of a P-L-I molecule could be measured by dissolving the P-L-I molecule in PBS pH 7.4 and after 24 h, investigate the ratio between P-Lp or Li-I and P-L-I using UPLC-MS.

In a preferred embodiment of the invention, either Lp or Li (or both Lp and Li) must be capable of binding covalently to carbohydrates, such as preferably glucose.

After binding to glucose, the respective fragments to which glucose is bound (P-Lp-Glc and/or Glc-Li-I) cannot any longer participate in the formation of the conjugate P-L-I. A compound is said to be able to bind covalently to glucose if it is capable of forming a glucose-conjugated structure within 72 hours of contacting the compound with a molar excess of glucose. The glucose binding capability of a linker *in vitro* can be measured by dissolving the linker (L) in PBS, 0.42 mM, pH 7.4 together with a 10000 eq. of glucose, and the generated Lp-Glc and/or Glc-Li is measured continuously for 72 h by LC-MS.

In an alternative embodiment, glucose may prevent the re-association of Lp and Li through another mechanism than binding to Lp or Li. In yet another alternative embodiment, glucose promotes, facilitates or enhances the hydrolysis of L.

In a preferred embodiment of the invention, the cyclic masked aldehyde or cyclic masked ketone in the linker (L) is a 5-membered ring. In yet a preferred embodiment of the invention, the cyclic masked aldehyde or cyclic masked ketone in the linker (L) comprises a moiety selected among O,O-acetals, N,O-acetals, N,N-acetals, S,N-acetals, S,O-acetals, S,S-acetals, O,O-ketals, N,O-ketals, N,N-ketals, S,N-ketals, S,O-ketals or S,S-ketals and their derivatives. In an embodiment of the invention, the masked aldehyde or masked ketone comprises a moiety selected among 1,3-dioxole, 2,3-dihydrooxazole (4-oxazoline), 2,3-dihydro-1*H*-imidazole (4-imidazoline), 2,3-dihydrothiazole (4-thiazoline), 1,3-oxathiole, 1,3-dithiole. In another embodiment of the invention, the masked aldehyde or masked ketone comprises a moiety selected among 2,5-dihydrooxazole (3-oxazole), 2,5-dihydro-1*H*-imidazole (3-imidazoline), 2,5-dihydrothiazole (3-thiazoline). In a preferred embodiment of the invention the masked aldehyde or masked ketone comprises a moiety selected among 1,3-dioxolane, oxazolidine, imidazolidine, thiazolidine, 1,3-oxathiolane or 1,3-dithiolane. In a highly preferred embodiment of the invention L comprises a 5-membered cyclic masked aldehyde. I yet another highly preferred embodiment, the 5-membered cyclic masked aldehyde is a thiazolidine, oxazolidine or a 1,3-dioxolane. We have observed that, compared to hydrolysable hydrazones and/or oximes known in prior art, 5-membered cyclic masked aldehydes and 5-membered cyclic masked ketones, in particular acetals, have the advantage of providing greater possibility to tune the stability and/or lability of the bond towards hydrolysis in the conjugates according to the invention, due to more positions that may be modified with e.g. EDG, EWG or bulky groups. In addition, acylhydrazones or hydrazones which are hydrolysed to hydrazides or hydrazines, may produce toxicity issues, which may be avoided using linkers comprising a 5-membered cyclic masked aldehyde or 5-membered cyclic masked ketone. In general, the acetal cleavage products are believed to be readily biologically degraded.

Thus, in an embodiment of the invention, L is of the general formulae I-III 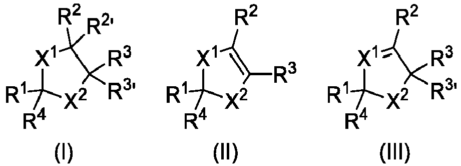 wherein
X¹ and X² is independently selected from NH, NR⁵, S or O
R¹ and/or R⁴ comprises P or I
R², R^{2'}, R³, R^{3'} and/or R⁵ comprises P or I, with the proviso that both P and I are present

In the present context, it should be understood that P and I should be comprised in the R groups (i.e. R¹⁻⁵) on opposite sides of the hydrolysable linker (i.e. such that they are cleaved upon hydrolysis into P-Lp and Li-I). It should further be understood that more than one P and more than one I may be present in the conjugate. Thus, in an embodiment of the invention, R¹ and/or R⁴ comprises I and R², R^{2'}, R³ and/or R^{3'} comprises P. In another embodiment of the invention, R¹ and/or R⁴ comprises P and R², R^{2'}, R³ and/or R^{3'} comprises I. In a preferred embodiment of the invention, R¹ comprises I and R³ or R^{3'} comprises P. In yet another preferred embodiment of the invention, R¹ comprises I and R² or R^{2'} comprises P. In still another preferred embodiment of the invention, R¹ comprises I and R⁵ comprises P.

The linkers of the general formula I-III may comprise up to three stereogenic centers in the core giving a possibility of eight stereoisomers, each of which will exist in an diastereoisomer or enantiomer relationship to each other. An enantiomer has the usual meaning in the art as one of two stereoisomers that are mirror images of each other that are non-superimposable. Stereoisomers that are not enantiomers to each other are diastereoisomers. In certain embodiments, wherein a symmetry plane exists in a stereoisomer, the mirror image of the stereoisomer is superimposable and thus the same compound, known as a meso isomer. It should be understood that the present invention is intended to include all individual stereoisomers possible as well as any mixtures thereof.

Thus, in an embodiment, the linker is a single stereoisomer. In yet another embodiment of the invention, the linker is a mixture of at two or more stereoisomers. In yet another embodiment, the linker may be a racemate (i.e. 1:1 of enantiomers). The linkers of the present invention may be synthesized e.g. by condensation between a non-chiral aldehyde and a chiral molecule (e.g. L-cysteine), leading to a scrambled chiral center on aldehyde carbon and a mixture of diastereoisomers. Diastereoisomers may have different stability/hydrolysability. In the event that a certain enantiomer or diastereoisomer is desired, conventional means known in the art, may be used for separation.

In a highly preferred embodiment of the invention according to the formulae I-III above, X¹ is NH and X² is S. In another preferred embodiment of this invention, X¹ is NH and X² is O. In another embodiment, X¹ is NH and X² is NH. In yet another embodiment of this invention, X¹ is NH and X² is NR⁵. In an even further embodiment of this invention, X¹ is S and X² is O. In another embodiment X¹ is S and X² is S. In yet another embodiment, X¹ is S and X² is NR⁵. In still another embodiment of this invention, X¹ is O and X² is O. In yet another embodiment of this invention, X¹ is O and X² is NR⁵.

### P-L-I:

The conjugate of the formula P-L-I according to the invention is a conjugate comprising the above-mentioned components P, L and I.

Due to the hydrolysable nature of L, the conjugate P-L-I exists *in vivo* in a dynamic equilibrium as exemplified in Figure 1, wherein P-L-I is in molar excess of one or both of P-Lₚ and Lᵢ-I. Due to the association of P and I, the conjugate P-L-I is inactive (or has a reduced efficacy) *in vivo,* whereas the peptide hormone P-Lₚ, as well as the peptide hormone P-Lₚ-Glc, is an active peptide hormone *in vivo.*

In a preferred embodiment of the invention, P-Lₚ or Lᵢ-I binds covalently to glucose (Glc), hence shifting the equilibrium towards hydrolysis of the linker (i.e. shifting the equilibrium towards peptide in high activation state).

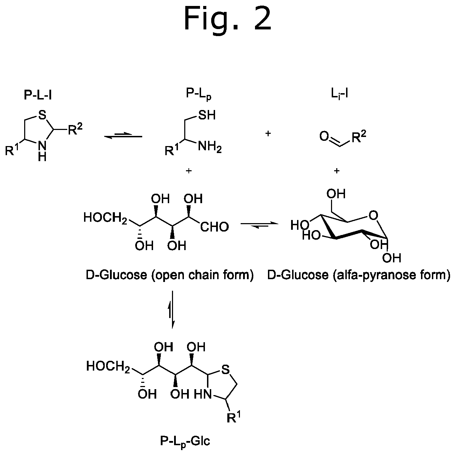

Thereby, activated P (P that is no longer associated with I) is then blocked from further associating with the inhibitor. As an example, if L comprises a thiazolidine, P-Lp comprises a 2-aminoethane-1-thiol and Li-I may comprise and aldehyde or vice versa. The 2-aminoethane-1-thiol in P-Lp may react with glucose to form a new thiazolidine as exemplified in Figure 2. Thereby, by binding to glucose, the 2-aminoethane-1-thiol of the active peptide hormone is blocked from reacting further with the inhibitor.

In theory, the P-Lₚ-Glc and/or I-LᵢGlc molecule is in an equilibrium with P-Lₚ and/or I-Lᵢ and glucose. However, as the glucose concentration is more than 10,000 equivalents higher than the P-Lₚ/I-Lᵢ part *in vivo,* the equilibrium is shifted towards P-Lₚ-Glc and/or I-Lᵢ-Glc. According to Le Châtelier's principle, rising glucose concentrations causes a shifting in the dynamic equilibrium, such that more peptide hormone (P) is released from the inhibitor and the equilibrium constant remains constant.

In an alternative embodiment of the invention, the hydrolysis of the hydrolysable linker L is promoted by glucose, whereby the dynamic equilibrium is altered in the presence of glucose.

In an embodiment of the invention, the conjugate is of the general formulae IV-VI 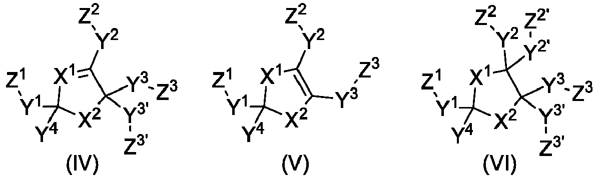 wherein
Z¹, Z², Z^{2'},Z³ and Z^{3'} is independently selected from P, I, hydrogen (-H) or not present, with the proviso that both P and I are present;
X¹ and X² is independently selected from N, NH, NR⁵, S or O;
Y¹ is selected from C₁-C₂₀ alkylene, C₂-C₂₀ alkenylene, C₂-C₂₀ alkynylene, a 5, 6, 9 or 10-membered aryl or heteroaryl, wherein said heteroaryl comprises 1-4 heteroatom(s) selected from N, O, or S, further wherein said Y¹ is optionally substituted with one or more substituents, which may be the same or different, and are independently selected from the group consisting of C₁-C₁₀ alkyl, C₂-C₁₀ alkenyl, C₂-C₁₀ alkynyl, phenyl (-Ph), -O-Ph, -NH-Ph, benzyl (-Bn), -O-Bn, -NH-Bn, benzoyl (Bz), -O-Bz, -NH-Bz, alkanolamines (-HN-(C₁-C₂₀-alkyl)-OH), (-HN-(C₁-C₂₀-alkyl)-NH₂), (-HN-(CH₂CH₂O)₁₋₂₀-H), amino (-NH₂), -CH₂NH(C₁-C₁₀ alkyl), -CH₂N(C₁-C₁₀ alkyl)₂, aminoalkyl (-NH(C₁-C₁₀ alkyl) or H₂N(C₁-C₁₀ alkyl)- or -N(C₁-C₁₀ alkyl)₂), cyano (-CN), -CONH₂, -CONH(C₁-C₂₅ alkyl), -CON(C₁-C₁₀ alkyl)₂, -NHCO(C₁-C₁₀ alkyl), hydroxyl (-OH), C₁-C₁₀ alkyl hydroxyl (-alkyl-OH), (-O-(C₁-C₂₀ alkyl)-OH), (-O-(C₁-C₂₀ alkyl)-NH₂), (-O-(CH₂CH₂O)₁₋₂₀-H), (-O-(C₁-C₂₀ alkyl)-COOH), (-NH-(C₁-C₂₀ alkyl)-COOH), (-NH-(C₁-C₂₀ alkyl)-*N*-(maleimide), (-O-(C₁-C₂₀ alkyl)-*N*-(maleimide), C₁-C₁₀ alkoxy(-O-alkyl), carboxylic acid (-COOH), C₁-C₁₀ alkyl esters (-COO-alkyl), C₁-C₁₀ alkyl acyl (-CO-alkyl), C₁-C₁₀ thioethers (-S-alkyl), sulfonic acid (-SO₃H), C₁-C₁₀ alkyl sulfonate (-SO₃-alkyl), phosphonic acid (-PO(OH)₂), C₁-C₁₀ alkyl phosphonate (-PO(O-alkyl)₂), phosphinic acid (-P(O)(H)OH), SO₂NH₂, hydroxamic acid (-CONHOH), alkyl sulfonylureas (-NHCONHSO₂(C₁-C₁₀ alkyl)), alkyl acylsulfonamides (-SO₂-NHCO-(C₁-C₁₀ alkyl), hydroxyl amine (-NHOH), nitro (-NO₂), and halogens; further wherein said alkylene, alkenylene, alkynylene optionally comprises one or more moieties within or in the end(s) of the chain, selected from the group consisting of ester (-COO-), ether (-O-), phenyl, amine (-NH-), amide (-CONH-), N-succinimide (-N(O₂H₃C₄)-), urea (-NHCONH-), thioamide (-C(S)NH-), carbamate (-NHC(O)O-), aldimine (-CH(N)-) or ketone (-CO-);
Y², Y^{2'}, Y³ and Y^{3'} is independently selected from hydrogen (-H), C₁-C₂₀ alkylene, C₂-C₂₀ C₂-C₂₀ alkenylene, C₂-C₂₀ alkynylene, wherein said C₁-C₂₀ alkylene, C₂-C₂₀ alkenylene, C₂-C₂₀ alkynylene comprises one or more moieties within or in the end(s) of the chain selected from the group consisting of ester (-COO-), ether (-O-), thioether (-S-), phenyl, amine (-NH- or -N(C₁-C₃ alkyl)-), amide (-CONH-), *N*-succinimide(-N(O₂H₃C₄)-), urea (-NHCONH-), thioamide (-C(S)NH-), carbamate (-NHC(O)O-), aldimine(-CH(N)-), or ketone (-CO-);
Y⁴ is selected from H, C₁-C₃ alkyl, phenyl, benzyl or benzoyl;
R⁵ is a C₁-C₅ alkyl or an amine protecting group such as Boc or Fmoc.

In the present context, an amine protecting group may serve the purpose of altering/tuning the hydrolysability of the acetal as well as providing chemo-selectivity during synthesis of the conjugates. Amine protecting groups are common known in the art as well as protection/deprotection conditions and may be found in Greene's Protective Groups in Organic synthesis.

In the present context, it should be understood that Y¹ may be covalently linked to Z¹ via one of the optional substituents on Y¹. Thus, in a highly preferred embodiment of the invention, Z¹ is linked to Y¹ via one of the optional substituents.

In an highly preferred embodiment, Y¹ is a 6-membered aryl (i.e. phenyl), substituted with one or more substituents selected from C₁-C₁₀ alkyl, -O-Ph, -NH-Ph, -O-Bn, -NH-Bn, -O-Bz, -NH-Bz, (-O-(C₁-C₂₀ alkyl)-NH₂), (-O-(C₁-C₂₀ alkyl)-OH), (-HN-(C₁-C₂₀-alkyl)-OH), (-HN-(C₁-C₂₀-alkyl)-NH₂), (-O-(CH₂CH₂O)₁-₂₀-H), (-HN-(CH₂CH₂O)₁₋₂₀-H), (-O-(C₁-C₂₀ alkyl)-COOH), (-NH-(C₁-C₂₀ alkyl)-COOH), (-NH-(C₁-C₂₀ alkyl)-*N*-(maleimide), (-O-(C₁-C₂₀ alkyl)-*N-*(maleimide), amino (-NH₂), aminoalkyl (-NH(C₁-C₁₀ alkyl) or -N(C₁-C₁₀ alkyl)₂), cyano (-CN), -CONH₂, -CONH(C₁-C₂₅ alkyl), -CON(C₁-C₁₀ alkyl)₂, -NHCO(C₁-C₁₀ alkyl), hydroxyl (-OH), C₁-C₁₀ alkoxy(-O-alkyl), carboxylic acid (-COOH), C₁-C₁₀ alkyl esters (-COO-alkyl), C₁-C₁₀ alkyl acyl (-CO-alkyl), nitro (-NO₂), and halogens.

In the most preferred embodiment, Y¹ is a 6-membered aryl (i.e. phenyl) substituted with one or more substituents selected from C₁-C₁₀ alkyl, -O-Ph, -NH-Ph, -O-Bn, -NH-Bn, -O-Bz, -NH-Bz, (-O-(C₁-C₂₀ alkyl)-NH₂), (-HN-(C₁-C₂₀-alkyl)-NH₂), (-O-(CH₂CH₂O)₁₋₂₀-H), (-HN-(CH₂CH₂O)₁₋₂₀-H), (-O-(C₁-C₂₀ alkyl)-COOH), (-NH-(C₁-C₂₀ alkyl)-COOH), (-NH-(C₁-C₂₀ alkyl)-*N*-(maleimide), (-O-(C₁-C₂₀ alkyl)-*N*-(maleimide), amino(-NH₂), aminoalkyl(-NH(C₁-C₁₀ alkyl) or -N(C₁-C₁₀ alkyl)₂), hydroxyl(-OH), C₁-C₁₀ alkoxy(-O-alkyl), nitro (-NO₂), and halogens.

In a highly preferred embodiment, Y¹ is a 6-membered aryl and is covalently linked to P via one of the optional substituents (-O-(C₁-C₂₀ alkyl)-NH₂), (-HN-(C₁-C₂₀-alkyl)-NH₂), (-O-(CH₂CH₂O)₁₋₂₀-H), (-HN-(CH₂CH₂O)₁₋₂₀-H), (-O-(C₁-C₂₀ alkyl)-COOH), (-NH-(C₁-C₂₀ alkyl)-COOH), (-NH-(C₁-C₂₀ alkyl)-*N*-(maleimide), (-O-(C₁-C₂₀ alkyl)-*N*-(maleimide) such that the conjugate comprises the moiety (-O-(C₁-C₂₀ alkyl)-NH-P), (-HN-(C₁-C₂₀-alkyl)-NH-P), (-O-(CH₂CH₂O)₁₋₂₀-P), (-HN-(CH₂CH₂O)₁₋₂₀-P), (-O-(C₁-C₂₀ alkyl)-CO-P), (-NH-(C₁-C₂₀ alkyl)-COP), (-NH-(C₁-C₂₀ alkyl)-*N*-(succinimide-P), (-O-(C₁-C₂₀ alkyl)-*N*-(succinimide-P). The succinimide linkage may be formed, by a 1,4-addition, between the maleimide and e.g. a cysteine in P.

In another highly preferred embodiment, Y¹ is a 6-membered aryl and is linked to I via one of the optional substituents (-O-(C₁-C₂₀ alkyl)-NH₂), (-HN-(C₁-C₂₀-alkyl)-NH₂), amino (-NH₂), aminoalkyl(-NH(C₁-C₁₀ alkyl), such that the conjugate comprises the moiety (-O-(C₁-C₂₀ alkyl)-NH-I), (-HN-(C₁-C₂₀-alkyl)-NH-I), amino(-NH-I), aminoalkyl(-N(I)(C₁-C₁₀ alkyl).

In a highly preferred embodiment, Y², Y^{2'}, Y³ and Y^{3'} are independently selected from hydrogen (-H), C₁-C₂₀ alkylene, wherein said C₁-C₂₀ alkylene comprises one or more moieties within or in the end(s) of the chain selected from the group consisting of ester (-COO-), ether (-O-), thioether (-S-), amine (-NH- or -N(C₁-C₃ alkyl)-), amide (-CONH-), N-succinimide (-N(O₂H₃C₄)-), or ketone (-CO-). In a preferred embodiment, one of Y², Y^{2'}, Y³ and Y^{3'} is covalently linked to P via a moiety in the end of the C₁-C₂₀ alkylene. As non-limiting examples, this may be achieved by e.g. reacting an activated carboxylic acid in the alkylene with a lysine residue in P, enzymatic transamidation between e.g. glutamine residue in P and an amine in the alkylene or by reacting a cysteine (i.e. thiol) in P with a maleimide in the alkylene to form a substituted succinimide.

In a preferred embodiment, Y² and Y^{2'} are H such that Z² and Z^{2'} are not present. In another preferred embodiment, Y³ and Y^{3'} are H such that Z² and Z^{2'} are not present.

In another highly preferred embodiment, Y⁴ is H, such that the linker (L) is an acetal.

In yet another highly preferred embodiment, P is linked to Y², Y^{2'}, Y³ or Y^{3'} via a succinimide. The succinimide linkage may be formed, by a 1,4-addition, between a maleimide and e.g. a cysteine in P.

In an embodiment of the invention, the conjugate is of the general formula (IV). In another embodiment of the invention the conjugate is of the general formula (V). In a highly preferred embodiment of the invention, the conjugate is of the general formula (VI)

In a highly preferred embodiment of the invention according to formulae IV - VI above, X¹ is S and X² is NH. In another highly preferred embodiment, X¹ is S and X² is NR⁵. In another preferred embodiment of this invention X¹ is O and X² is NH. In yet another preferred embodiment of this invention, X¹ is O and X² is O. In another embodiment X¹ is NH and X² is NH. In yet another embodiment of this invention, X¹ is NH and X² is NR⁵. In an even further embodiment of this invention X¹ is S and X² is O. In another embodiment X¹ is S and X² is S. In yet another embodiment of this invention, X¹ is O and X² is NR⁴. In another embodiment of the invention, X¹ is S and X² is N. In yet another embodiment of the invention, X¹ is NH and X² is N. In even another embodiment of the invention, X¹ is O and X² is N.

In a highly preferred embodiment of the invention, the conjugate is of the general formula (VI) and X¹ is S and X² is NH. In yet a highly preferred embodiment, the conjugate is of the general formula (VI) X¹ is S and X² is NR⁵.

In a preferred embodiment of the invention, the conjugate is of the general formula (VII). In another preferred embodiment of the invention, the conjugate is of the general formula (VIII). In a highly preferred embodiment of the invention, the conjugate is of the general formulae (IX).

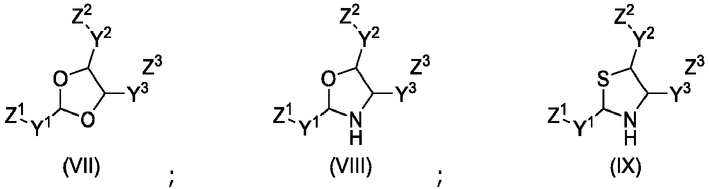

In a second and highly preferred aspect, the present invention relates to the use of a conjugate, according to the first aspect, for use in the treatment of a medical condition in a human being or animal subject. Thus, the present invention relates to the conjugate, according to the first aspect, for use in the treatment of a medical condition in a human being or animal subject. In the present context a medical condition is a broad term that includes all diseases and disorders. The term disease (e.g. diabetes mellitus) has the usual meaning in the art and should be understood as any condition which results in the disorder of a structure or function in a living organism that is not due to any external injury. The term disorder (e.g. a metabolic disorder) has the usual meaning in the art and should be understood as a functional abnormality or disturbance (e.g. hyperglycemia). Treatment should be understood in the broadest sense as prevention, amelioration or treatment of one or more medical conditions. Thus, the conjugates, according to the present invention, may also be used for prophylactic treatment of a human or animal subject.

In particular, the conjugates according to the present invention may be used for the treatment of diabetes mellitus in a human or animal subject. In a highly preferred embodiment, the present invention relates to the use thereof in the treatment of a human subject. Even more particularly, the conjugates according to the present invention may be used for the treatment of diabetes mellitus in a human or animal subject, the treatment comprising administering the conjugate in a frequency of 2 or less administrations per day. Even more particularly, the conjugates according to the present invention may be used for the treatment of diabetes mellitus in a human or animal subject, the treatment comprising administering the conjugate in a frequency of 1 or less administrations per day. A frequency of 1 or less administration per day should be understood as for example 1 administration every second day, such as 1 administration every third day, such as for example 1 administration once a week. The conjugates of the present invention may be administered by any suitable route known in the art. Typical routes of administration may include oral, pulmonary or parenteral administration, preferably parenteral. Parenteral administration includes intravenous, intramuscular, subcutaneous and intradermal, preferably subcutaneous administration.
Thus, the present invention also relates to a method of treatment of diabetes mellitus, said method comprising administering the conjugate according to the invention to a person in need thereof.

In a third aspect, the invention relates to a pharmaceutical composition comprising a conjugate according to the first aspect, and at least one pharmaceutically acceptable carrier and/or excipient. Thus, in a highly preferred embodiment, the present invention relates to the use a conjugate according to the first aspect, in the manufacture of a medicament for the treatment of diabetes in a human subject. In an embodiment of the invention, the pharmaceutical composition is intended for veterinary use. In highly preferred embodiment of the invention, the pharmaceutical composition is intended for use in a human subject.

### EXAMPLES

Example 1-3 exemplifies the synthesis of linker (L) molecules.
Example 4 exemplifies the synthesis of a linkers attached to an inhibitor (L-I)
Example 5 exemplifies the synthesis of insulin conjugates according to the invention
Example 6 exemplifies the analyses of the linker (L) molecules for their ability to hydrolyse *in vitro.*
Example 7 exemplifies the *in vitro* insulin receptor B (INSRb) functional assay
Example 1: Synthesis of exemplary linkers - thiazolidines

### Benzyl N-(tert-butoxycarbonyl)-S-trityl-L-cysteinate

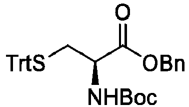

Potassium carbonate (3.46g, 25.0 mmol) and benzyl bromide (2.05 g, 12.0 mmol) were added to a solution of N-(tert-butoxycarbonyl)-S-trityl-L-cysteine (4.64 g, 10.0 mmol) in 40 mL acetone. The reaction mixture was refluxed overnight. After evaporation of the solvent, the residue was partitioned between 40 mL water and 2x200 mL diethyl ether, the combined organic phases were dried over sodium sulfate and evaporated. The residue was purified by chromatography on silica gel using petroleum ether:ethyl acetate 4:1 as the eluent affording the title compound as a light yellow oil (4.88 g, 88%). ¹H NMR (400 MHz, DMSO-d6) δ 7.40 (d, J = 8.2 Hz, 1H), 7.36 - 7.18 (m, 20H), 5.09 - 4.95 (m, 2H), 3.83 (td, J = 9.3, 4.8 Hz, 1H), 2.65 - 2.54 (m, 1H), 2.36 (dd, J = 12.4, 4.7 Hz, 1H), 1.36 (s, 9H).

### (R)-1-(benzyloxy)-3-mercapto-1-oxopropan-2-aminium trifluoroacetate

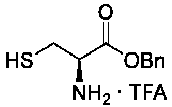

Benzyl N-(tert-butoxycarbonyl)-S-trityl-L-cysteinate (4.8 g, 8.7 mmol) was treated with 100 mL of a 94:3:3 mixture of trifluoroacetic acid:water:triisopropylsilane and stirred for 1 h at room temperature. The white precipitate was removed by filtration and the filtrate was evaporated at 35 °C. The crude product was dissolved in 3.5 mL acetonitrile and 2.5 mL water and purified on a Biotage SNAP C18 column using a gradient of acetonitrile in water containing 0.1% trifluoroacetic acid as the eluent. Freeze drying afforded the title compound (1.75g, 62%). ¹H NMR (400 MHz, DMSO-d6) δ 8.52 (s,broad, 3H), 7.47 - 7.33 (m, 5H), 5.26 (s, 2H), 4.46 (t, J = 4.7 Hz, 1H), 3.01 (d, J = 4.8 Hz, 2H).

### General procedure thiazolidines:

(R)-1-(benzyloxy)-3-mercapto-1-oxopropan-2-aminium trifluoroacetate (1 equiv) was dissolved in dioxane:ethanol 1:1 and added to the aldehyde (1 equiv) and N,N-diisopropylethylamine (1 equiv) in dioxane:ethanol under argon atmosphere. The reaction mixture was heated to 50°C overnight, evaporated and then purified by chromatography on a Biotage KP-SIL column using a gradient of 0-100 % ethyl acetate in petroleum ether as the eluent to give the title compound.

### Linker 1.

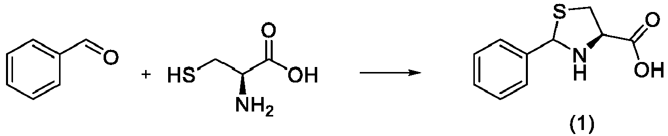

### Linker 2.

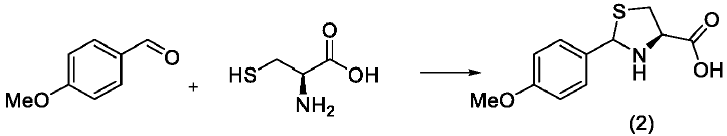

### Linker 3.

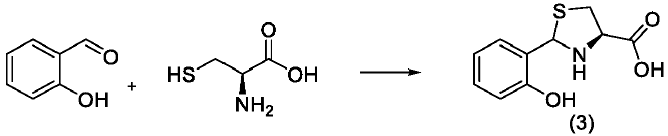

### Linker 4.

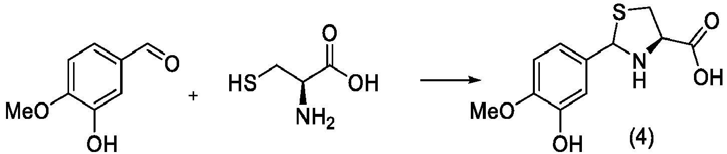

### Linker 5.

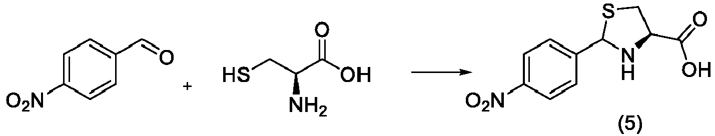

### Linker 6.

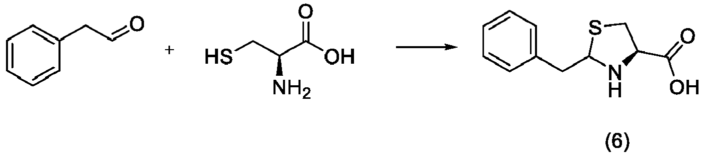

### Linker 7. Benzyl (4R)-2-phenyl-1,3-thiazolidine-4-carboxylic acid

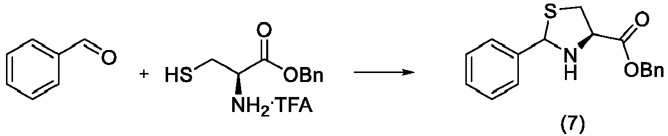

Linker 7 was synthesised according to the general thiazolidine procedure using benzaldehyde as starting material. ¹H NMR (400 MHz, Chloroform-d) δ 7.51 (dd, J = 16.4, 7.4 Hz, 2H), 7.44 - 7.28 (m, 8H), 5.88 - 5.53 (m, 1H), 5.31 - 5.18 (m, 2H), 4.33 - 3.98 (m, 1H), 3.45 (ddd, J = 28.3, 10.1, 7.4 Hz, 1H), 3.28 - 3.07 (m, 1H), 2.94 - 2.61 (m, 1H). MS (ES+) m/z 300 [M+H]⁺.

### Linker 8. Benzyl (4R)-2-(4-methoxyphenyl)-1,3-thiazolidine-4-carboxylic acid

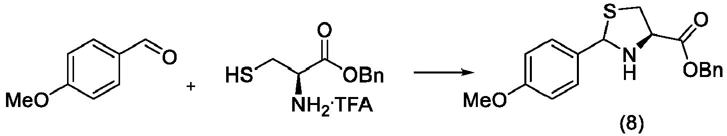

Linker 8 was synthesised according to the general thiazolidine procedure using p-anisaldehyde as starting material. ¹H NMR (400 MHz, Chloroform-d) δ 7.49 - 7.31 (m, 7H), 6.88 (dd, J = 14.1, 8.3 Hz, 2H), 5.82 - 5.50 (m, 1H), 5.29 - 5.17 (m, 2H), 4.33 - 3.97 (m, 1H), 3.81 (dd, J = 6.8, 0.8 Hz, 3H), 3.44 (ddd, J = 23.9, 10.4, 7.6 Hz, 1H), 3.27 - 3.08 (m, 1H), 2.64 (s, 1H). MS (ES+) m/z 330 [M+H]⁺.

### Linker 9. Benzyl (4R)-2-(3-methoxyphenyl)-1,3-thiazolidine-4-carboxylic acid

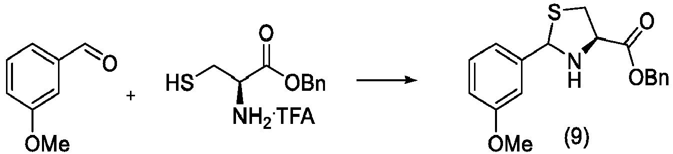

Linker 9 was synthesised according to the general thiazolidine procedure using m-anisaldehyde as starting material. ¹H NMR (400 MHz, Chloroform-d) δ 7.46 - 7.21 (m, 6H), 7.16 - 7.03 (m, 2H), 6.85 (ddd, J = 27.7, 8.2, 2.4 Hz, 1H), 5.87 - 5.49 (m, 1H), 5.31 - 5.16 (m, 2H), 4.31 - 3.95 (m, 1H), 3.82 (d, J = 5.8 Hz, 3H), 3.44 (ddd, J = 27.4, 10.4, 7.1 Hz, 1H), 3.25 - 3.07 (m, 1H), 2.96 - 2.61 (m, 1H). MS (ES+) m/z 330 [M+H]⁺.

### Linker 10. Benzyl (4R)-2-(2-methoxyphenyl)-1,3-thiazolidine-4-carboxylic acid

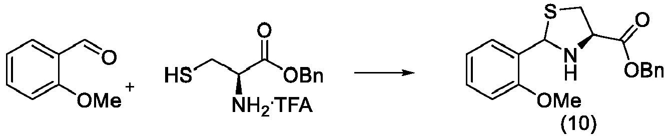

Linker 10 was synthesised according to the general thiazolidine procedure using o-anisaldehyde as starting material. ¹H NMR (400 MHz, Chloroform-d) δ 7.56 - 7.19 (m, 7H), 6.93 (ddt, J = 20.1, 16.4, 7.9 Hz, 2H), 6.09 - 5.81 (m, 1H), 5.32 - 5.18 (m, 2H), 4.37 - 3.93 (m, 1H), 3.87 (s, 3H), 3.40 (ddd, J = 46.6, 10.3, 6.8 Hz, 1H), 3.24 - 2.98 (m, 2H). MS (ES+) m/z 330 [M+H]⁺.

### Linker 11. Benzyl (4R)-2-(4-acetamidophenyl)-1,3-thiazolidine-4-carboxylic acid

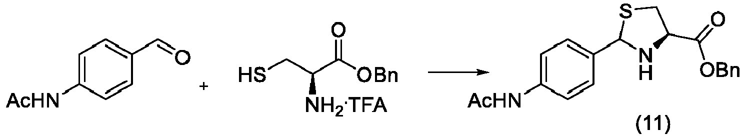

Linker 11 was synthesised according to the general thiazolidine procedure using 4-acetatamidobenzaldehyde as staring material. ¹H NMR (400 MHz, Chloroform-d) δ 7.58 - 7.31 (m, 8H), 7.15 (d, J = 13.1 Hz, 1H), 5.86 - 5.47 (m, 1H), 5.24 (d, J = 6.5 Hz, 2H), 4.32 - 3.95 (m, 1H), 3.44 (ddd, J = 29.9, 10.4, 7.2 Hz, 1H), 3.29 - 3.05 (m, 1H), 2.92 - 2.56 (m, 1H), 2.18 (d, J = 4.9 Hz, 3H). MS (ES+) m/z 357 [M+H]⁺.

### Linker 12. Benzyl (4R)-2-(3-acetamidophenyl)-1,3-thiazolidine-4-carboxylic acid

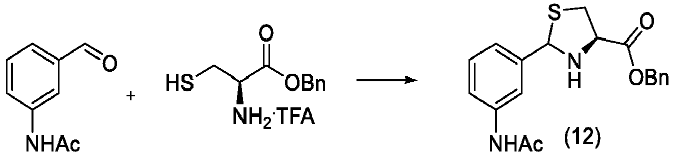

Linker 12 was synthesised according to the general thiazolidine procedure using 3-acetatamidobenzaldehyde as staring material. ¹H NMR (400 MHz, Chloroform-d) δ 7.65 - 7.46 (m, 2H), 7.44 - 7.28 (m, 6H), 7.23 (m, 2H), 5.83-5.51 (m, 1H), 5.24 (d, J = 5.9 Hz, 2H), 4.27 - 3.97 (m, 1H), 3.44 (ddd, J = 29.4, 10.5, 7.1 Hz, 1H), 3.23 - 3.07 (m, 1H), 2.98-2.58 (m, 1H), 2.18 (d, J = 4.8 Hz, 3H). MS (ES+) m/z 357 [M+H]⁺.

### Example 2: Synthesis of exemplary linkers - oxazolidines

### General procedure oxazolidines:

The aldehyde (1 equiv) and N,N-diisopropylethylamine (1 equiv) were added to benzyl L-serinate hydrochloride (1 equiv) and magnesium sulfate (2 equiv) in tetrahydrofuran and the reaction was stirred at 50°C overnight. The reaction mixture was filtered through a pad of Celite, washed with tetrahydrofuran and the volatiles were removed under reduced pressure. The residue was dissolved in acetonitrile, triethylamine (2 equiv) and di-tert-butyl dicarbonate (1.1 equiv) were added, and the resulting mixture was stirred overnight. The reaction mixture was concentrated, and the residue purified on a Biotage KP-SIL column manually eluted with 5% ethyl acetate in petroleum ether followed by 10% ethyl acetate in petroleum ether affording the title compound.

### Linker 13. Benzyl 3-tert-butyl (4S)-2-(4-methoxyphenyl)-1,3-oxazolidine-4-carboxylic acid

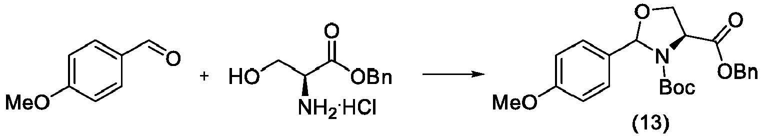

Linker 13 was synthesised according to the general oxazolidine procedure using p-anisaldehyde as starting material. ¹H NMR (400 MHz, DMSO-d6) δ 7.52 - 7.29 (m, 7H), 6.83 (d, J = 8.5 Hz, 2H), 5.81 (d, J = 42.1 Hz, 1H), 5.22 (d, J = 6.0 Hz, 2H), 4.69 - 4.58 (m, 1H), 4.22 (s, 2H), 3.74 (s, 3H), 1.20 (d,J=61.4 Hz, 9H). MS (ES+) m/z 436 [M+Na]⁺.

### Linker 14. Benzyl 3-tert-butyl (4S)-2-(4-acetamidophenyl)-1,3-oxazolidine-4-carboxylic acid

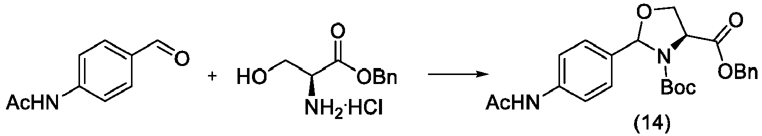

Linker 14 was synthesised according to the general oxazolidine procedure using 4-acetatamidobenzaldehyde as staring material.

### Linker 15. Benzyl 3-tert-butyl (4S)-2-phenyl-1,3-oxazolidine-4-carboxylic acid

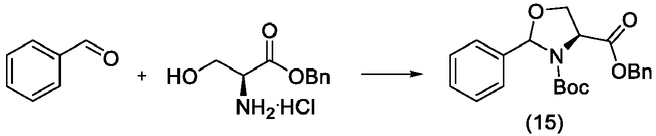

Linker 15 was synthesised according to the general oxazolidine procedure using benzaldehyde as starting material. ¹H NMR (400 MHz, DMSO-d6) δ 7.55 (d, J = 6.9 Hz, 2H), 7.44 - 7.25 (m, 8H), 5.86 (d, J = 45.3 Hz, 1H), 5.23 (s, 2H), 4.69 - 4.62 (m, 1H), 4.19 (d, J = 43.6 Hz, 2H), 1.18 (d, J = 76.7 Hz, 9H). MS (ES+) m/z 406 [M+Na]⁺.

### Example 3: Synthesis of exemplary linkers - dioxolanes

### General procedure dioxolanes:

The aldehyde (1.2 equiv), trimethyl orthoformate (1.2 equiv) and p-toluenesulfonic acid monohydrate (0.05 equiv) were added to (+)-dimethyl L-tartrate (1.2 equiv) in toluene. The mixture was heated to 70°C for 3 hours 45 minutes, allowing volatiles to evaporate. The reaction mixture was attained room temperature and the reaction mixture was purified on a Biotage KP-SIL column using a gradient of 0-100% ethyl acetate in petroleum ether as the eluent affording the title compound.

### Linker 16. 4,5-Dimethyl (4R,5R)-2-phenyl-1,3-dioxolane-4,5-dicarboxylate

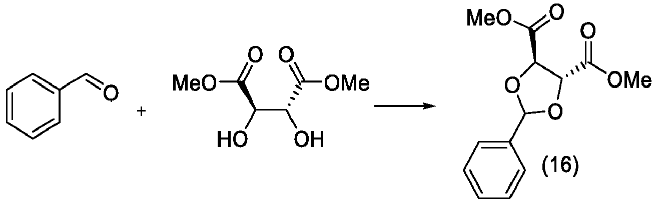

Linker 16 was synthesised according to the general dioxolanes procedure using benzaldehyde as starting material. ¹H NMR (400 MHz, Chloroform-d) δ 7.62 - 7.55 (m, 2H), 7.44 - 7.37 (m, 3H), 6.15 (s, 1H), 4.99 (d, J = 4.0 Hz, 1H), 4.88 (d, J = 4.0 Hz, 1H), 3.89 (d, J = 0.9 Hz, 3H), 3.84 (d, J = 0.9 Hz, 3H). MS (ES+) m/z 267 [M+H]+.

### Linker 17. 4,5-Dimethyl (4R,5R)-2-(4-methoxyphenyl-1,3-dioxolane-4,5-dicarboxylate

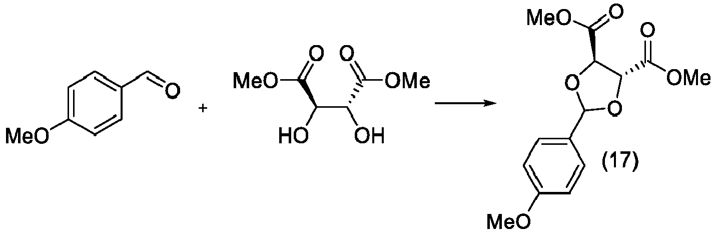

Linker 17 was synthesised according to the general dioxolanes procedure using p-anisaldehyde as starting material. ¹H NMR (400 MHz, Chloroform-d) δ 7.52 (d, J = 8.7 Hz, 2H), 6.93 (d, J = 8.7 Hz, 2H), 6.11 (s, 1H), 4.97 (d, J = 4.0 Hz, 1H), 4.85 (d, J = 4.0 Hz, 1H), 3.90 - 3.80 (m, 9H). MS (ES+) m/z 297 [M+H]⁺.

### Linker 18. 4,5-Dimethyl (4R,5R)-2-(3-methoxyphenyl-1,3-dioxolane-4,5-dicarboxylate

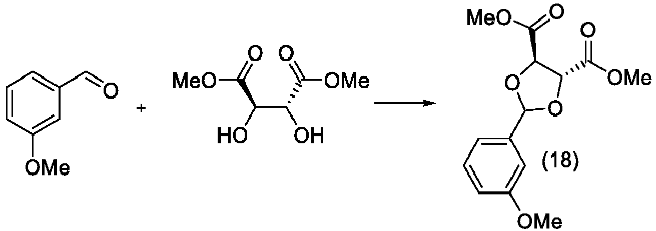

Linker 18 was synthesised according to the general dioxolanes procedure using m-anisaldehyde as starting material. ¹H NMR (400 MHz, Chloroform-d) δ 7.32 (t, J = 7.8 Hz, 1H), 7.20 - 7.13 (m, 2H), 6.95 (dd, J = 8.2, 2.6 Hz, 1H), 6.14 (s, 1H), 4.99 (d, J = 4.0 Hz, 1H), 4.87 (d, J = 4.0 Hz, 1H), 3.89 (s, 3H), 3.84 (s, 6H). MS (ES+) m/z 297 [M+H]⁺.

### Linker 19. 4,5-Dimethyl (4R,5R)-2-(2-methoxyphenyl-1,3-dioxolane-4,5-dicarboxylate

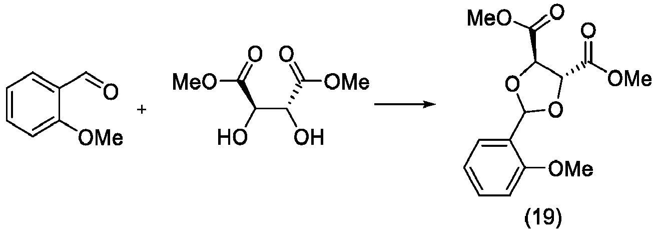

Linker 19 was synthesised according to the general dioxolanes procedure using o-anisaldehyde as starting material. ¹H NMR (400 MHz, Chloroform-d) δ 7.69 (d, J = 7.6 Hz, 1H), 7.40 - 7.33 (m, 1H), 7.01 (s, 1H), 6.91 (d, J = 8.3 Hz, 1H), 6.54 (s, 1H), 5.01 (d, J = 4.0 Hz, 1H), 4.88 (d, J = 4.0 Hz, 1H), 3.87 (d, J = 6.3 Hz, 6H), 3.81 (s, 3H). MS (ES+) m/z 297 [M+H]⁺.

### Linker 20. 4,5-Dimethyl (4R,5R)-2-(4-acetamidophenyl-1,3-dioxolane-4,5-dicarboxylate

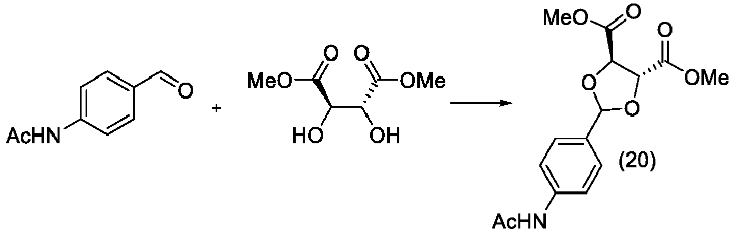

Linker 20 was synthesised according to the general dioxolanes procedure using 4-acetamidobenzldehyde as starting material. ¹H NMR (400 MHz, Chloroform-d) δ 7.55 (s, 4H), 7.24 (s, 1H), 6.11 (s, 1H), 4.97 (d, J = 4.0 Hz, 1H), 4.86 (d, J = 4.0 Hz, 1H), 3.86 (d, J = 16.1 Hz, 6H), 2.19 (s, 3H). MS (ES+) m/z 324 [M+H]⁺.

### Linker 21. 4,5-Dimethyl (4R,5R)-2-(3-acetamidophenyl-1,3-dioxolane-4,5-dicarboxylate

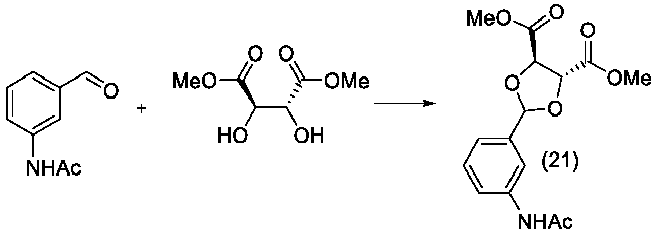

Linker 21 was synthesised according to the general dioxolanes procedure using 3-acetamidobenzldehyde as starting material. ¹H NMR (400 MHz, Chloroform-d) δ 7.67 (d, J = 9.3 Hz, 2H), 7.34 (d, J = 16.2 Hz, 2H), 6.12 (s, 1H), 4.97 (d, J = 4.0 Hz, 1H), 4.87 (dd, J = 4.0, 1.1 Hz, 1H), 3.88 (d, J = 1.2 Hz, 3H), 3.84 (d, J = 1.2 Hz, 3H), 2.18 (s, 3H). MS (ES+) m/z 324 [M+H]⁺.

### Linker 22. 4,5-Dimethyl (4R,5R)-2-(2-acetamidophenyl-1,3-dioxolane-4,5-dicarboxylate

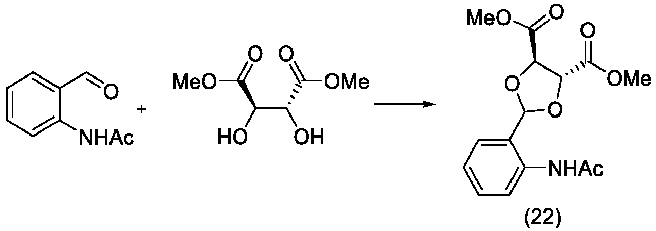

Linker 22 was synthesised according to the general dioxolanes procedure using 2-acetamidobenzldehyde as starting material. ¹H NMR (400 MHz, Chloroform-d) δ 8.69 (s, 1H), 8.25 (d, J = 8.0 Hz, 1H), 7.43 (t, J = 8.2 Hz, 2H), 7.13 (t, J = 7.3 Hz, 1H), 6.04 (s, 1H), 4.91 - 4.83 (m, 2H), 3.91 (d, J = 0.8 Hz, 3H), 3.87 (d, J = 0.8 Hz, 3H), 2.24 (s, 3H). MS (ES+) m/z 324 [M+H]⁺.

### Example 4: Synthesis of an exemplary hvdrolvsable linkers attached to an inhibitor (L-I)

### N-[4-(hydroxymethyl)phenyl]octadecanamide

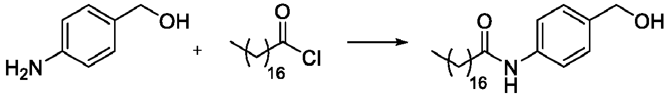

To a solution of (4-aminophenyl)methanol (1.00 g, 8.12 mmol) in 20 mL pyridine, was added stearoyl chloride (2.74 mL, 8.12 mmol) dropwise during 10 minutes, and the resulting slurry was stirred overnight. The reaction mixture was poured into 500 mL water, the precipitate was collected by filtration and washed with water. Co-evaporation with acetonitrile and drying under vacuum gave the title compound as a solid (2.90 g, 92%). ¹H NMR (400 MHz, Chloroform-d) δ 7.55 - 7.48 (m, 2H), 7.36 - 7.30 (m, 2H), 4.87 (d, J = 162.5 Hz, 2H), 2.35 (q, J = 8.5 Hz, 2H), 1.79 - 1.62 (m, 2H), 1.26 (s, 28H), 0.89 (t, J = 6.6 Hz, 3H).

### N-[4-(formyl)phenyl]octadecanamide

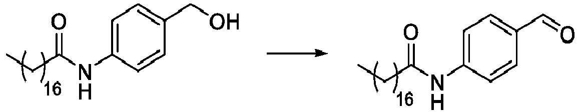

N-[4-(hydroxymethyl)phenyl]octadecanamide (0.39 g, 1.00 mmol) was dissolved in 20 mL dichloromethane and then manganese(IV)oxide (0.87 g, 10.00 mmol) was added. The reaction mixture was stirred overnight, filtered through Celite and the filtrate was evaporated to give the title compound as a white solid (0.18 g, 48%). ¹H NMR (400 MHz, Chloroform-d) δ 9.93 (s, 1H), 7.86 (d, J = 8.4 Hz, 2H), 7.71 (d, J = 8.5 Hz, 2H), 2.41 (t, J = 7.5 Hz, 2H), 1.75 (m, 2H), 1.26 (m, 28H), 0.89 (t, J = 6.7 Hz, 3H).

### Linker-inhibitor 1. (4R)-2-(4-stearamidophenyl)thiazolidine-4-carboxylic acid

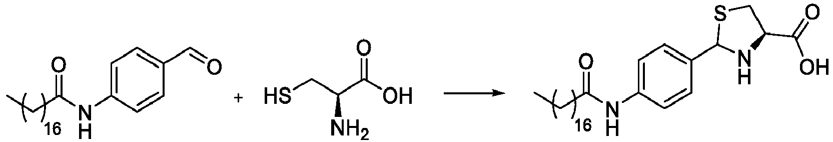

To N-(4-formylphenyl)octadecanamide (0.15 g, 0.40 mmol) and L-cysteine (63.0 mg, 0.52 mmol) under argon, was added 4 mL of a degassed mixture of methylene chloride:methanol 1:1, and the resulting mixture was heated to 50°C for 3 days. More L-cysteine (14.5 mg, 0.12 mmol) was added and the mixture was stirred at 50°C overnight. The reaction mixture was allowed to cool to room temperature, and the solid was collected by filtration, after washing with water, the title compound was obtained (98 mg, 49%). ¹H NMR (400 MHz, DMSO-d6) δ 9.88 (d, J = 18.2 Hz, 1H), 7.55 (dd, J = 16.5, 8.1 Hz, 2H), 7.42 (d, J = 8.1 Hz, 1H), 7.34 (d, J = 8.3 Hz, 1H), 5.52 (d, J = 64.6 Hz, 1H), 4.27 - 3.80 (m, 1H), 3.18 - 3.00 (m, 2H), 2.26 (d, J = 7.8 Hz, 2H), 1.57 (s, 2H), 1.23 (m, 28H), 0.85 (t, J = 6.4 Hz, 3H). MS (ES+) m/z 491 [M+H]⁺.

### Example 5: Synthesis of insulin conjugate according to the invention

### General procedure:

Dimethylformamide (0.5 mL) and 15-crown-5 (10 equivalents) were added to (4R)-2-(4-stearamidophenyl)thiazolidine-4-carboxylic acid (1 mg) and stirred for 1 h until the starting materials were dissolved. TSTU (O-(N-succinimidyl)-1,1,3,3-tetramethyl uranium tetrafluoroborate) (1.3 equivalents) in dimethylformamide (0.1 mL) was added and the reaction was stirred at room temperature for 10 min, followed by dropwise addition to a gently stirred solution of human insulin (2 equivalents) in DMSO containing Et₃N (100 equivalents). After 10 min, the reaction mixture was analyzed by LC-MS confirming the formation of the insulin conjugate. Acetonitrile (0.5 mL) and water (0.5 mL) were added and pH was adjusted to 7.5 by addition of acetic acid. Purification by RP-Flash Chromatography (Isolera One™, Biotage) on a 10 g C4 column using a gradient of water, 0.1% formic acid towards acetonitrile 0.1% formic acid. The pH of each fraction was adjusted to around 7.5 using aqueous NH₃. The pure fractions were pooled, acetonitrile was evaporated and pH was adjusted to 8 by aqueous NH₃, followed by lyophilized to give the insulin conjugate as white powder.

### Example 6: Analysis of the linkers in example 1-3 for their ability to undergo hydrolysis with or without glucose present

### General protocol:

1.3-1.8 mg of linkers was dissolved in 100 µL DMSO or 100 µL MeCN. 10 µL was taken out from the DMSO stock solution, diluted with 90µL DMSO and dissolved in 900 µL 1xPBS buffer containing 10 000 equiv glucose, pH 7.4 or 900 µL 1xPBS buffer pH 7.4, to give a final concentration of 0.42 mM of the linker. 10 µL was taken out from the MeCN stock solution, diluted with 40 µL MeCN and dissolved in 950 µL 1xPBS buffer containing 10 000 equiv glucose, pH 7.4 or 950 µL 1xPBS buffer pH 7.4 to give a final concentration of 0.42 mM of the linker. The samples were incubated at 37°C and continuously analysed by LC-MS or UPLC.

### Half-life of the linker

| Linker | 5% MeCN in PBS buffer, 10.000 equiv Glc (h) | 10% DMSO in PBS buffer, 10.000 equiv Glc (h) | 5% MeCN in PBS buffer, no Glc (h) | 10% DMSO in PBS buffer, no Glc (h) |
|---|---|---|---|---|
| 1 | n.a | 12 | n.a | >48 |
| 2 | n.a | 2 | n.a | 4 |
| 3 | n.a | 0.5 | n.a | 2 |
| 4 | n.a | 4 | n.a | 10 |
| 5 | n.a | >24 | n.a | >72 |
| 6 | n.a | 24 | n.a | >72 |
| 7 | >48 | >48 | n.a | n.a |
| 8 | 46 | 42 | n.a | n.a |
| 9 | >72 | >72 | n.a | n.a |
| 10 | 45 | 23 | n.a | n.a |
| 11 | >72 | >72 | n.a | n.a |
| 12 | n.a | n.a | n.a | n.a |
| 13 | >72 | >72 | >72 | >72 |
| 14 | n.a | n.a | n.a | n.a |
| 15 | >72 | >72 | >72 | >72 |
| 16 | n.a | n.a | n.a | n.a |
| 17 | 2.2 | 1.1 | 2.2 | 1.8 |
| 18 | n.a | n.a | n.a | n.a |
| 19 | 2.5 | 1.5 | 2.5 | 1.7 |
| 20 | n.a | n.a | n.a | n.a |
| 21 | n.a | n.a | n.a | n.a |
| 22 | n.a | n.a | n.a | n.a |

### Example 7: In vitro insulin receptor B (INSRb) functional assay

The purpose of this example is to show how to test the *in vitro* potency of conjugates according to the invention on the insulin B receptor. For this purpose, reference conjugates comprising a hydrazine linker, without inhibitor (R1) and with inhibitor (R2) was used.

### General protocol:

The PathHunter INSRb functional assay kit (DiscoverX) with a 1 x PBS buffer containing 0.1% BSA (Bovine Serum Albumin) pH 7.4, instead of the manufacturing buffer, was used.

| **Compound** | **EC50 (nM)** |
|---|---|
| Human insulin | 0.10 |
| Insulin conjugate R1 (hydrazone reference) | 0.11 |
| Insulin-B29-C18 | 31 |
| Insulin conjugate R2 (hydrazone reference) | 8.2 |

### Result and discussion:

The potency of the reference insulin conjugate R1(insulin without inhibitor) is similar to the potency of human insulin. The potency of the insulin conjugate R2 is 100-fold lower than that of human insulin and the potency of insulin-C18 is 300-fold lower than the potency of human insulin.

## Claims

1. A conjugate of the formula P-L-I, wherein P is a peptide hormone effecting the metabolism of carbohydrates *in vivo,* L is a hydrolysable linker molecule consisting of Lₚ and Lᵢ, and I is a C₁₂-C₂₆ lipid molecule capable of inactivating or inhibiting the effect of the peptide hormone P on the metabolism of carbohydrates *in vivo,* **characterised in that**
a. the linker molecule L comprises a 5-membered cyclic masked aldehyde or a 5-membered cyclic masked ketone hydrolysable *in vivo,* such that the conjugate P-L-I and the conjugate parts P-Lₚ and Lᵢ-I exist in a dynamic equilibrium *in vivo* where the conjugate P-L-I exists in molar excess of at least one of the conjugate parts P-Lₚ and Lᵢ-I, and further **characterised in that**
b. at least one of the conjugate parts P-Lₚ and Lᵢ-I binds covalently to glucose, whereby the concentration of P that is not bound to I increases *in vivo* when the concentration of glucose increases *in vivo,* or, alternatively further **characterised in that** the hydrolysis of the hydrolysable linker L is being promoted by glucose.

2. Conjugate according to claim 1, wherein the reactant P-Lₚ binds covalently to glucose.

3. Conjugate according to claims 1 and 2, wherein P is insulin or an insulin analogue.

4. Conjugate according to anyone of the preceding claims, wherein I is an agent capable of inhibiting the active site of P.

5. Conjugate according to anyone of the preceding claims, wherein I is an agent capable of clustering multiple conjugates of the formula P-L-I *in vivo.*

6. Conjugate according to anyone of the preceding claims, wherein I is an agent capable of binding to serum albumin.

7. Conjugate according to anyone of the preceding claims, wherein the masked aldehyde or masked ketone comprises a group selected among O,O-acetals, N,O-acetals, N,N-acetals, S,N-acetals, S,O-acetals, S,S-acetals, O,O-ketals, N,O-ketals, N,N-ketals, S,N-ketals, S,O-ketals or S,S-ketals and their derivatives.

8. Conjugate according to anyone of the preceding claims, wherein L is of the general formulae I-III 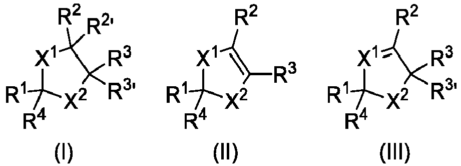 wherein
X¹ and X² are independently selected from N, NH, NR⁵, S or O
R¹ and/or R⁴ comprises P or I
R², R^{2'}, R³, R^{3'} and/or R⁵ comprises P or I, with the proviso that both P and I are present

9. Conjugate according to anyone of the preceding claims, of the general formulae IV-VI 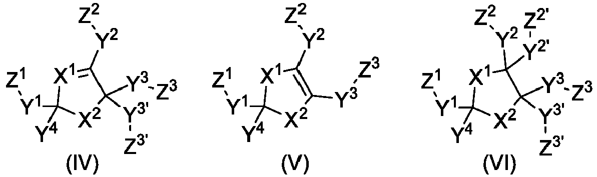 wherein
Z¹, Z², Z^{2'}, Z³ and Z^{3'} is independently selected from P, I, hydrogen (-H) or not present, with the proviso that both P and I are present;
X¹ and X² are independently selected from N, NH, NR⁵, S or O;
Y¹ is selected from C₁-C₂₀ alkylene, C₂-C₂₀ alkenylene, C₂-C₂₀ alkynylene, a 5, 6, 9 or 10-membered aryl or heteroaryl, wherein said heteroaryl comprises 1-4 heteroatom(s) selected from N, O, or S, further wherein said Y¹ is optionally substituted with one or more substituents, which may be the same or different, and are independently selected from the group consisting of C₁-C₁₀ alkyl, C₂-C₁₀ alkenyl, C₂-C₁₀ alkynyl, phenyl (-Ph), -O-Ph, -NH-Ph, benzyl (-Bn), -O-Bn, -NH-Bn, benzoyl (Bz), -O-Bz, -NH-Bz, alkanolamines (-HN-(C₁-C₂₀-alkyl)-OH), (-HN-(C₁-C₂₀-alkyl)-NH₂), (-HN-(CH₂CH₂O)₁₋₂₀-H), amino (-NH₂), -CH₂NH(C₁-C₁₀ alkyl), -CH₂N(C₁-C₁₀ alkyl)₂, aminoalkyl(-NH(C₁-C₁₀ alkyl) or H₂N(C₁-C₁₀ alkyl)- or -N(C₁-C₁₀ alkyl)₂), cyano (-CN), -CONH₂, -CONH(C₁-C₂₅ alkyl), -CON(C₁-C₁₀ alkyl)₂, -NHCO(C₁-C₁₀ alkyl), hydroxyl (-OH), C₁-C₁₀ alkyl hydroxyl (-alkyl-OH), (-O-(C₁-C₂₀ alkyl)-OH), (-O-(C₁-C₂₀ alkyl)-NH₂), (-O-(CH₂CH₂O)₁₋₂₀-H), (-O-(C₁-C₂₀ alkyl)-COOH), (-NH-(C₁-C₂₀ alkyl)-COOH), (-NH-(C₁-C₂₀ alkyl)-*N*-(maleimide), (-O-(C₁-C₂₀ alkyl)-*N*-(maleimide), C₁-C₁₀ alkoxy(-O-alkyl), carboxylic acid (-COOH), C₁-C₁₀ alkyl esters (-COO-alkyl), C₁-C₁₀ alkyl acyl (-CO-alkyl), C₁-C₁₀ thioethers (-S-alkyl), sulfonic acid (-SO₃H), C₁-C₁₀ alkyl sulfonate (-SO₃-alkyl), phosphonic acid (-PO(OH)₂), C₁-C₁₀ alkyl phosphonate (-PO(O-alkyl)₂), phosphinic acid (-P(O)(H)OH), SO₂NH₂, hydroxamic acid (-CONHOH), alkyl sulfonylureas (-NHCONHSO₂(C₁-C₁₀ alkyl)), alkyl acylsulfonamides (-SO₂-NHCO-(C₁-C₁₀ alkyl), hydroxyl amine (-NHOH), nitro (-NO₂), and halogens; further wherein said alkylene, alkenylene, alkynylene optionally comprises one or more moieties within or in the end(s) of the chain, selected from the group consisting of ester (-COO-), ether (-O-), phenyl, amine (-NH-), amide (-CONH-), N-succinimide (-N(O₂H₃C₄)-), urea (-NHCONH-), thioamide (-C(S)NH-), carbamate (-NHC(O)O-), aldimine (-CH(N)-) or ketone (-CO-);
Y², Y^{2'}, Y³ and Y^{3'} is independently selected from hydrogen (-H), C₁-C₂₀ alkylene, C₂-C₂₀ C₂-C₂₀ alkenylene, C₂-C₂₀ alkynylene, wherein said C₁-C₂₀ alkylene, C₂-C₂₀ alkenylene, C₂-C₂₀ alkynylene comprises one or more moieties within or in the end(s) of the chain selected from the group consisting of ester (-COO-), ether (-O-), thioether (-S-), phenyl, amine (-NH- or -N(C₁-C₃ alkyl)-), amide (-CONH-), *N*-succinimide (-N(O₂H₃C₄)-), urea (-NHCONH-), thioamide (-C(S)NH-), carbamate (-NHC(O)O-), aldimine (-CH(N)-), or ketone (-CO-);
Y⁴ is selected from H, C₁-C₃ alkyl, phenyl, benzyl or benzoyl;
R⁵ is a C₁-C₅ alkyl or an amine protecting group such as Boc or Fmoc.

10. Conjugate according to anyone of the preceding claims, wherein I is selected among 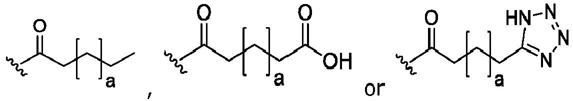 wherein a is an integer from 8-22.

11. Conjugate according to anyone of the preceding claims, wherein the general formula is formula (VI)

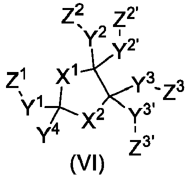

12. Conjugate according to anyone of the preceding claims, for use as a medicament.

13. Conjugate according to anyone of the preceding claims, for use in the treatment of diabetes in a human or animal subject.

14. Conjugate according to claim 12, for use in the treatment of diabetes mellitus in a human or animal subject, the treatment comprising administering the conjugate in a frequency of 1 or less administrations per day.

15. Pharmaceutical or veterinary composition comprising a conjugate according to any of claims 1-11 and at least one pharmaceutically acceptable carrier and/or excipient.
